# EUROPEAN PATENT APPLICATION

(11) **EP 4 830 732 A1**
(43) Date of publication of application: **28.10.2026**
(21) Application number: 24906384.3
(22) Date of filing: 18.12.2024
(51) Int. Cl.: A61K 47/68, C07K 16/28, A61K 31/4745, A61K 31/40, A61P 35/00, C12N 15/63

(54) **ANTI-FGFR2B ANTIBODY-DRUG CONJUGATE AND USE THEREOF**

(30) Priority: 18.12.2023 CN 202311755620; 11.12.2024 CN 202411822694
(71) Applicant: Shandong Simcere Zaiming Biopharmaceutical Co., Ltd., Yantai, Shandong 264006 (CN)
(72) Inventor: FU, Yayuan, Shanghai 201318 (CN); GE, Yuanjie, Shanghai 201318 (CN); TANG, Renhong, Shanghai 201318 (CN)
(74) Representative: Müller-Boré & Partner Patentanwälte PartG mbB
(86) International application number: PCT/CN2024/140216
(87) International publication number: WO 2025/130905

(57) **Abstract**

An antibody-drug conjugate (ADC) targeting human FGFR2 and use thereof, particularly an ADC specifically binding to a human FGFR2b isoform, having relatively strong killing activity against tumor cells endogenously expressing FGFR2b, such as KATO III and SNU-16, and especially exhibiting a relatively high tumor inhibition rate in an in-vivo efficacy model.

## Description

The present disclosure claims the priority to Chinese Patent Application No. 202311755620.1 entitled "ANTI-FGFR2B ANTIBODY-DRUG CONJUGATE AND USE THEREOF", filed on December 18, 2023, and Chinese Patent Application No. 202411822694.7 entitled "ANTI-FGFR2B ANTIBODY-DRUG CONJUGATE AND USE THEREOF", filed on December 11, 2024 with China National Intellectual Property Administration, which are incorporated herein by reference in their entirety.

### TECHNICAL FIELD

The present disclosure relates to the field of antibodies, and in particular, to an anti-FGFR2b antibody-drug conjugate and use thereof.

### BACKGROUND

The fibroblast growth factor (FGF) family consists of 22 known FGFs, and is divided into 7 subfamilies based on the activity and sequence similarity. Only 4 fibroblast growth factor receptors (FGFR1-4) and their subtypes are currently known to bind to FGFs. The combination of FGFRs with the ligand and heparin induces FGFRs to form a dimer, autophosphorylating the tyrosine kinase domain in cells, activating a number of signal transduction pathways in cells such as proliferation (STATs, RAS/p38/JNKs, and RAS/MAPK/ERK), survival (STATs and PI3K/AKT), cytoskeleton regulation (PLC/Ca²⁺), and the like, and promoting cell proliferation, survival, and differentiation. Therefore, FGFRs can participate in regulating physiological processes in organisms such as embryonic development, organ development, wound healing, angiogenesis, and the like. Different subtypes may have different ligands, while FGFR2b is a receptor with high affinity for the FGF7 subfamily. Different subtypes also differ in their expression in tissues. FGFR2b is mainly expressed in epithelial tissues (such as epithelial cells on the surface of colorectal mucosa, epithelial cells from the esophageal basal cells to the lower one third, epithelial cells on the surface of gastric mucosa, squamous epithelium on the surface of endometrium, vascular smooth muscle cells, and the like), while FGFR2c is mainly expressed in interstitial tissues.

The overexpression and mutation of FGFRs may lead to abnormalities in the FGFR signaling pathway, and thus promote the development and progression of tumors by promoting cell proliferation, survival, migration, and angiogenesis. FGFR2 overexpression and mutation are found in a variety of solid tumors. FGFR2b subtype expression can be found in gastric cancer, squamous cell lung cancer, triple-negative breast cancer, endometrial carcinoma, ovarian cancer, pancreatic cancer, intrahepatic cholangiocarcinoma, and colorectal cancer. Gastric cancer is a common malignant tumor worldwide. It affects more than one million patients annually and ranks third in mortality, just behind lung cancer and breast cancer. However, the therapies for gastric cancer are limited, and especially for HER2-negative patients, who constitute a considerable proportion, there's a lack of effective therapies. Statistical data revealed that about 30% of patients with HER2-negative advanced gastric cancer and gastroesophageal junction cancer are FGFR2b-positive. It is confirmed by immunohistochemical staining, sequencing, and other means that FGFR2b subtype, rather than FGFR2c subtype, is specifically overexpressed in gastric cancer tissues, while no FGFR2b expression is detected in peripheral tissues or normal organs (except skin), indicating that FGFR2b can be used as a treatment target in such gastric cancer patients. In addition, FGFR2b is highly expressed (2+/3+ IHC) in 31% of squamous cell lung cancers, 13% of triple-negative breast cancers, 40% of ovarian cancers, 4% of pancreatic cancers, and 22% of intrahepatic cholangiocarcinomas.

Antibody-drug conjugates (ADCs) are a novel tumor treatment consisting of an antibody for selectively recognizing surface antigens of cancer cells, a drug payload, and a linker. By conjugating an antibody targeting a specific antigen to a cytotoxic drug, the treatment integrates the tumor-targeting property of the antibodies with the potent killing effect of conventional small-molecule chemotherapeutics, and can precisely attack tumor cells and reduce damage to normal cells.

### SUMMARY

In one aspect, the present disclosure provides an antibody-drug conjugate having a structural general formula of Pc-(L-D)ₙ or a pharmaceutically acceptable salt thereof, wherein
D is a cytotoxic drug;
L is a linker unit;
Pc is an antibody or an antigen-binding fragment thereof that specifically binds to FGFR2b; and
n is a real number of 1 to 16.

In some embodiments, the antibody or the antigen-binding fragment thereof has one or more of the following properties:
(1) binding to FGFR2 with an affinity of no more than 5 × 10⁻⁸ M;
(2) binding to human, murine, or monkey FGFR2; and
(3) binding to FGFR2b but not to FGFR2c.

In some embodiments, the antibody or the antigen-binding fragment thereof comprises a heavy chain variable region (VH) or/and a light chain variable region (VL), wherein the heavy chain variable region comprises an HCDR1, an HCDR2, and an HCDR3, or/and the light chain variable region comprises an LCDR1, an LCDR2, and an LCDR3, wherein the HCDR1-3 or/and the LCDR1-3 are selected from the following combinations:
(1) the HCDR1-3 comprise the sequences set forth in SEQ ID NOs. 9-11, or/and the LCDR1-3 comprise the sequences set forth in SEQ ID NOs. 12-14;
(2) the HCDR1-3 comprise the sequences set forth in SEQ ID NOs. 15-17, or/and the LCDR1-3 comprise the sequences set forth in SEQ ID NOs. 18-20;
(3) the HCDR1-3 comprise the sequences set forth in SEQ ID NOs. 21-23, or/and the LCDR1-3 comprise the sequences set forth in SEQ ID NOs. 24-26; or
(4) the HCDR1-3 or/and the LCDR1-3 comprise sequences having at least 80% identity or having at most 3 insertion, deletion, or substitution mutations compared to any one CDR in the HCDR1-3 and the LCDR1-3 in any one of combinations (1)-(3); preferably, the at least 80% identity is 85%, 90%, 91%, 92%, 93%, 94%, 95%, 96%, 97%, 98%, 99%, or 100% identity.

In some embodiments, the HCDRs and LCDRs of the antibody or the antigen-binding fragment thereof are defined according to the Kabat numbering scheme.

In some embodiments, the antibody or the antigen-binding fragment thereof comprises a heavy chain variable region (VH) or/and a light chain variable region (VL), wherein the heavy chain variable region or/and the light chain variable region are selected from the following:
(1) the heavy chain variable region comprises the sequence set forth in SEQ ID NO. 1, or/and the light chain variable region comprises the sequence set forth in SEQ ID NO. 2;
(2) the heavy chain variable region comprises the sequence set forth in SEQ ID NO. 3, or/and the light chain variable region comprises the sequence set forth in SEQ ID NO. 4;
(3) the heavy chain variable region comprises the sequence set forth in SEQ ID NO. 5, or/and the light chain variable region comprises the sequence set forth in SEQ ID NO. 6; or
(4) the heavy chain variable region or/and the light chain variable region comprise sequences having at least 80% identity or having at most 3 insertion, deletion, or substitution mutations compared to the heavy chain variable region or/and the light chain variable region in any one of combinations (1)-(3); preferably, the at least 80% identity is 85%, 90%, 91%, 92%, 93%, 94%, 95%, 96%, 97%, 98%, 99%, or 100% identity.

In some embodiments, the antibody or the antigen-binding fragment thereof comprises a heavy chain constant region sequence and/or a light chain constant region sequence; optionally, the heavy chain constant region and/or the light chain constant region are selected from an intact constant region sequence or a fragment thereof, and the constant region fragment comprises CH1, a hinge region, CH2, CH3, or Fc; optionally, the heavy chain constant region is selected from a human or murine IgG1, IgG2, IgG3, or IgG4 constant region, and the light chain constant region is selected from a human or murine κ constant region or λ constant region; optionally, the antibody or the antigen-binding fragment thereof comprises intact heavy and light chains, wherein the heavy chain consists of the VH and the heavy chain constant region, the heavy chain constant region having the sequence set forth in SEQ ID NO: 7, and the light chain consists of the VL and the light chain constant region, the light chain constant region having the sequence set forth in SEQ ID NO: 8.

In some embodiments, the antibody or the antigen-binding fragment thereof is: (1) a chimeric antibody or a fragment thereof, (2) a humanized antibody or a fragment thereof, and/or (3) a fully human antibody or a fragment thereof.

Preferably, the antibody or the antigen-binding fragment thereof is selected from a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, a naked antibody, a conjugated antibody, a humanized antibody, a fully human antibody, a Fab, a Fab', an F(ab')₂, an Fd, an Fv, an scFv, a diabody, or a single domain antibody.

In some embodiments, the antigen-binding fragment is selected from one or more of an F(ab)₂, a Fab', a Fab, an Fv fragment, an scFv, a nanobody, or an affibody.

In some embodiments, the cytotoxic drug is selected from a tubulin inhibitor, a DNA damaging agent, or a topoisomerase inhibitor, wherein the tubulin inhibitor includes, but is not limited to, a dolastatin, an auristatin, a maytansine, a tubulysin, and a cryptomycin, the DNA damaging agent includes, but is not limited to, a PBD drug, and the topoisomerase inhibitor includes, but is not limited to, a camptothecin drug.

In some embodiments, the cytotoxic drug is selected from a topoisomerase I inhibitor or an auristatin drug.

In some embodiments, the cytotoxic drug is selected from a compound of formula (D-I), 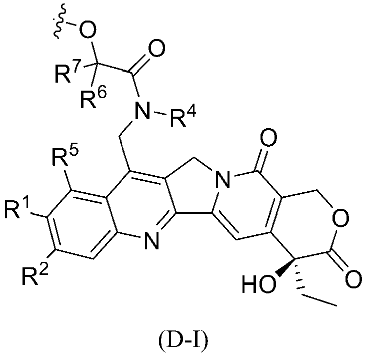 wherein
R¹ and R², together with the atoms linked thereto, form a 5- to 6-membered heterocyclic ring, wherein the 5- to 6-membered heterocyclic ring comprises 1 or 2 oxygen atoms as ring atoms, and the 5- to 6-membered heterocyclic ring is optionally substituted with one or more deuterium atoms;
R⁴ is selected from H or C₁-C₃ alkyl;
R⁵ is selected from H, halogen, CN, =O, OH, NH₂, or C₁-C₃ alkyl;
R⁶ is selected from H or C₁-C₃ alkyl;
R⁷ is selected from H, C₁-C₃ alkyl, or C₃-C₆ cycloalkyl, wherein the C₁-C₃ alkyl or the C₃-C₆ cycloalkyl is optionally substituted with deuterium, halogen, CN, =O, OH, NH₂, or C₁-C₃ alkyl.

In some embodiments, R¹ and R², together with the atoms linked thereto, form 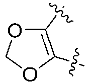 or

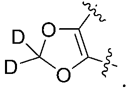

In some embodiments, R⁴ is selected from H.

In some embodiments, R⁵ is selected from H.

In some embodiments, R⁶ is selected from H.

In some embodiments, R⁷ is selected from cyclopropyl.

In some embodiments, the compound of formula (D-I) is selected from

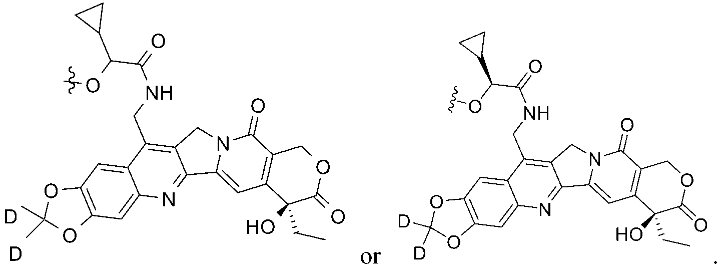

In some embodiments, the cytotoxic drug is selected from

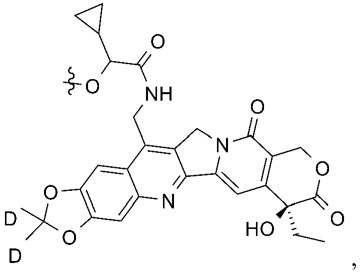

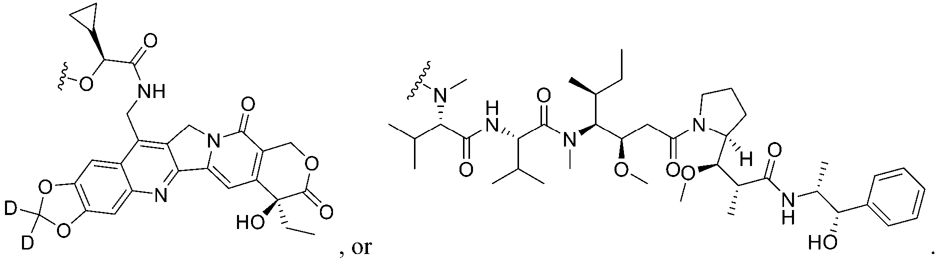

In some embodiments, the linker unit is selected from 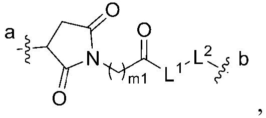 wherein m1 is selected from integers of 2 to 8, L¹ is selected from a peptide residue composed of 1 to 8 amino acids, and the peptide residue is further optionally substituted with one or more substituents selected from halogen, CN, =O, C₁-C₆ alkyl, OH, O(C₁-C₆ alkyl), NH₂, NH(C₁-C₆ alkyl), N(C₁-C₆ alkyl)₂, C₃-C₆ cycloalkyl, and 4- to 7-membered heterocyclyl; L² is selected from 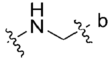 or 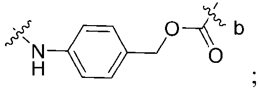 the end a of the linker unit is covalently linked to the antibody or the antigen-binding fragment thereof, and the end b is covalently linked to the cytotoxic drug.

In some embodiments, L¹ is selected from the peptide residue set forth in Val-Cit or Gly-Gly-Phe-Gly.

In some embodiments, m1 is 5.

In some embodiments, the linker unit is 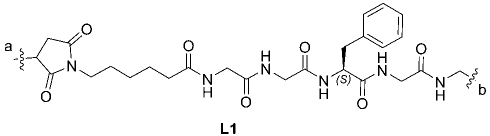 or 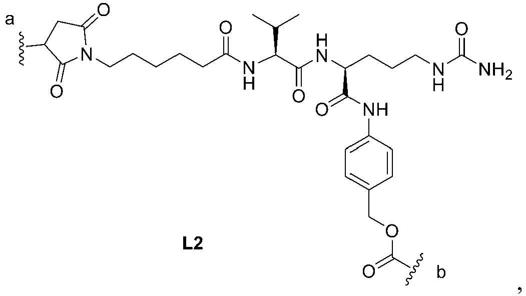 the end a of the linker unit is covalently linked to the antibody or the antigen-binding fragment thereof, and the end b of the linker unit is covalently linked to the cytotoxic drug.

In some embodiments, n is selected from real numbers of 1 to 16; for example, n is selected from real numbers of 2 to 12; for example, n is selected from real numbers of 4 to 10; for example, n is selected from real numbers of 3 to 9; for example, n is selected from real numbers of 4 to 8; for example, n is selected from real numbers of 6 to 8; for example, n is selected from real numbers of 3 to 5.

In some embodiments, n is selected from real numbers of 3 to 9; for example, n is 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0.

In some embodiments, n is selected from real numbers of 6 to 8; for example, n is 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0.

In some embodiments, n is selected from real numbers of 3 to 5; for example, n is 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0.

In some embodiments, the antibody-drug conjugate or the pharmaceutically acceptable salt thereof is selected from the following compounds or pharmaceutically acceptable salts thereof: 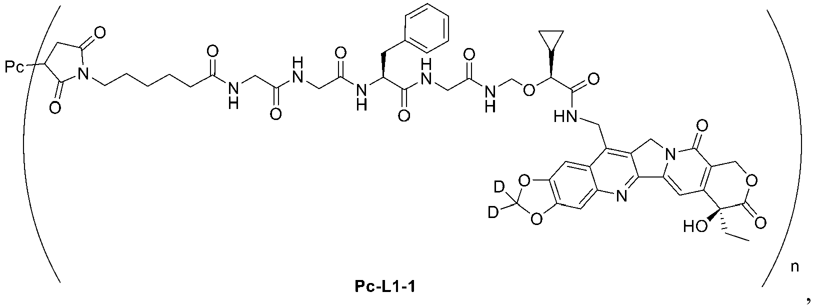 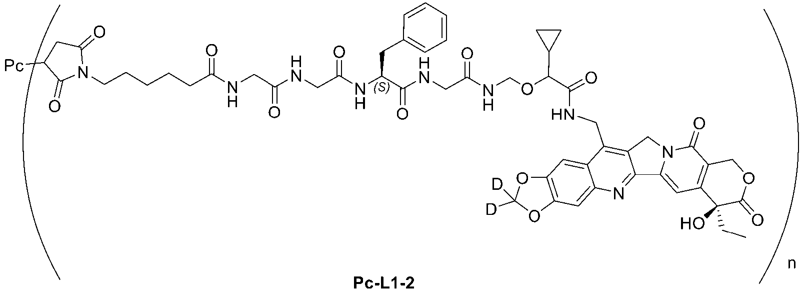 or 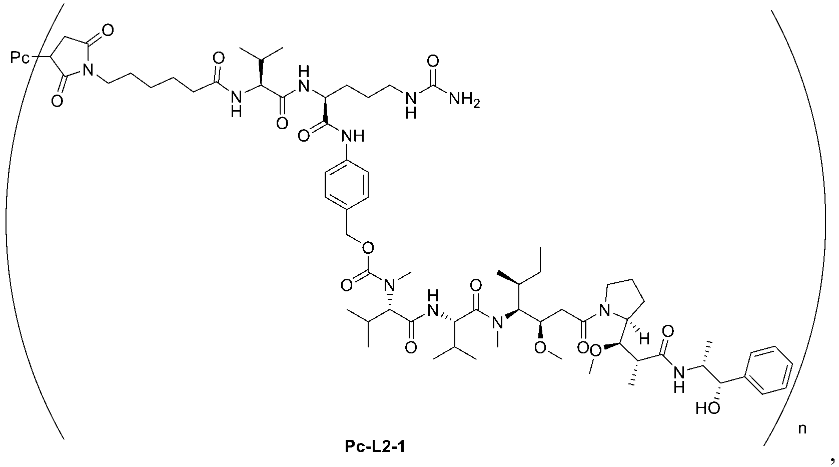 wherein Pc and n are as defined in any one of the preceding embodiments.

In another aspect, the present disclosure provides an isolated nucleic acid molecule, wherein the nucleic acid molecule may be in the form of isolated nucleotides, deoxynucleotides, and/or ribonucleotides in any length, and the nucleic acid molecule encodes the antibody or the antigen-binding fragment thereof described above.

In some embodiments, the present disclosure provides an expression vector comprising the nucleic acid molecule described above.

In some embodiments, the present disclosure provides an isolated host cell comprising the nucleic acid molecule described above or the expression vector described above; preferably, the host cell is a eukaryotic cell or a prokaryotic cell; more preferably, the host cell is derived from a mammalian cell, a yeast cell, an insect cell, an *Escherichia coli* cell, and/or a *Bacillus subtilis* cell; more preferably, the host cell is selected from an Expi293 cell or a CHO cell.

In another aspect, the present disclosure provides a pharmaceutical composition comprising the antibody-drug conjugate having the general formula of Pc-(L-D)ₙ or the pharmaceutically acceptable salt thereof described above in the present disclosure, and a pharmaceutically acceptable excipient.

In some embodiments, the pharmaceutical composition further comprises an additional therapeutic agent, such as an additional antibody, typically including a PD-1/PD-L1 inhibitor, or such as a chemotherapeutic agent.

In another aspect, the present disclosure provides a method for treating a tumor in a mammal, comprising administering to the mammal, preferably a human, in need of the treatment a therapeutically effective amount of the antibody-drug conjugate having the general formula of Pc-(L-D)n or the pharmaceutically acceptable salt thereof or the pharmaceutical composition described above.

In some embodiments, the method further comprises use of an additional therapeutic agent, such as an additional antibody, typically including a PD-1/PD-L1 inhibitor, or such as a chemotherapeutic agent.

In some embodiments, the tumor is an FGFR2b-expressing tumor.

In some embodiments, the tumor is selected from the group consisting of: gastric cancer, squamous cell lung cancer, triple-negative breast cancer, endometrial carcinoma, ovarian cancer, pancreatic cancer, intrahepatic cholangiocarcinoma, and colorectal cancer.

In another aspect, the present disclosure provides use of the antibody-drug conjugate having the general formula of Pc-(L-D)ₙ or the pharmaceutically acceptable salt thereof described above or the pharmaceutical composition described above in preparing a medicament for treating a tumor.

In some embodiments, the pharmaceutical composition further comprises an additional therapeutic agent, such as an additional antibody, typically including a PD-1/PD-L1 inhibitor, or such as a chemotherapeutic agent.

In some embodiments, the tumor is an FGFR2b-expressing tumor.

In some embodiments, the tumor is selected from the group consisting of: gastric cancer, squamous cell lung cancer, triple-negative breast cancer, endometrial carcinoma, ovarian cancer, pancreatic cancer, intrahepatic cholangiocarcinoma, and colorectal cancer.

In another aspect, the present disclosure provides use of the antibody-drug conjugate having the general formula of Pc-(L-D)ₙ or the pharmaceutically acceptable salt thereof described above or the pharmaceutical composition described above in treating a tumor.

In some embodiments, the pharmaceutical composition further comprises an additional therapeutic agent, such as an additional antibody, typically including a PD-1/PD-L1 inhibitor, or such as a chemotherapeutic agent.

In some embodiments, the tumor is an FGFR2b-expressing tumor.

In some embodiments, the tumor is selected from the group consisting of: gastric cancer, squamous cell lung cancer, triple-negative breast cancer, endometrial carcinoma, ovarian cancer, pancreatic cancer, intrahepatic cholangiocarcinoma, and colorectal cancer.

In another aspect, the present disclosure provides the antibody-drug conjugate having the general formula of Pc-(L-D)ₙ or the pharmaceutically acceptable salt thereof described above or the pharmaceutical composition thereof for use in treating a tumor.

### Beneficial Effects:

The antibody-drug conjugate of the present disclosure has the following advantages:
1. The antibody-drug conjugate can specifically recognize the FGFR2b target and exhibits higher selectivity for FGFR2b compared with FGFR2c;
2. Compared with the existing monoclonal antibody therapies, the antibody-drug conjugate has better anti-tumor effects and broad clinical application prospects in the treatment of diseases such as tumors.
3. As of the completion of the present disclosure, no ADC drugs targeting FGFR2b have entered the clinical stage. Through research, the inventors have obtained an ADC against FGFR2b, which has efficacy superior to that of bemarituzumab at equivalent or lower doses. Moreover, through verification, it has been found that the FGFR2b-ADC of the present disclosure can significantly inhibit tumor growth in a mouse xenograft model of a bemarituzumab-resistant gastric cancer patient-derived tumor.

### BRIEF DESCRIPTION OF THE DRAWINGS

FIG. 1 illustrates the binding activity of antibodies and ADCs to tumor cells;
FIG. 2 illustrates the inhibitory effects of ADCs on the *in vitro* proliferation of tumor cells;
FIG. 3 illustrates the regulation of SNU-16-#232 tumor growth by ADCs and the body weight profiles of tumor-bearing mice;
FIG. 4 illustrates the regulation of KATOIII-#729 tumor growth by ADCs and the body weight profiles of tumor-bearing mice;
FIG. 5 illustrates the comparison of the binding activity to tumor cells with reference antibodies and ADCs; and
FIG. 6 illustrates the comparison of the inhibitory effects on the *in vitro* proliferation of tumor cells with reference ADCs.

### DETAILED DESCRIPTION

The present disclosure will be further described with reference to specific examples, and the advantages and features of the present disclosure will become more apparent with the description. Experimental procedures without specified conditions in the examples are conducted according to conventional conditions or conditions recommended by the manufacturers. Reagents or instruments without specified manufacturers used herein are conventional products that are commercially available.

The examples are exemplary only and do not limit the scope of the present disclosure in any way. It will be appreciated by those skilled in the art that various changes or substitutions in form and details may be made to the technical solutions of the present disclosure without departing from the spirit and scope of the present disclosure, and that these changes and substitutions shall all fall within the scope of the present disclosure.

### Terminology and Definitions

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be interpreted according to its common meaning in the field. When referring to a trade name, it is intended to refer to its corresponding commercial product or its active ingredient.

Furthermore, unless otherwise stated herein, terms used in the singular form herein shall include the plural form, and vice versa. More specifically, as used in this specification and the claims, unless otherwise clearly indicated, the singular forms "a", "an", and "the" include referents in the plural form.

The terms "including", "comprising", and "having" herein are used interchangeably and are intended to indicate the inclusive nature of a solution, implying that there may be elements other than those listed in the solution. Meanwhile, it will be appreciated that the descriptions "including", "comprising", and "having" as used herein also provide the embodiments of "consisting of ... ".

The term "and/or" as used herein includes the meanings of "and", "or", and "all or any other combination of elements linked by the term".

The term FGFR (fibroblast growth factor receptor) generally includes four types: FGFR1-FGFR4 (also referred to as CD331-334). FGFRs are a subfamily of the tyrosinase receptor family, and are type I transmembrane proteins comprising: an extracellular domain comprising a ligand-binding site consisting of 2 or 3 immunoglobulin-like domains (IgI to IgIII); a single-pass membrane domain; and an intracellular domain comprising a tyrosine kinase domain. FGFRs generally function as a dimer. The extracellular domain comprises three Ig-like regions D1 (IgI), D2 (IgII), and D3 (IgIII). D1 and the acidic box constitute a self-inhibitory region; D2 and D3 are responsible for ligand binding (D2 binds to cell surface heparan sulfate, D3 includes IIIb and IIIc forms differentiated by alternative splicing, and only IIIc is found in FGFR4 currently). FGFRs can be divided into two forms according to the number of Ig-like domains, wherein one form is α type comprising three regions: IgI, IgII, and IgIII; the other is β type, comprising only IgII and IgIII. Exemplarily, the FGFR2b in the present disclosure includes "FGFR2(α)b" and "FGFR2(β)b".

The human FGF family includes 22 FGFs (FGF1 to FGF14 and FGF16 to FGF23). Except that FGF19, FGF21, and FGF23 are produced in endocrine means, the other members are produced by paracrine means. FGF7 can only bind to type IIIb FGFR2. The binding of FGFRs to FGFs mediates the activation and transduction of RAS-RAF-MAPK, PI3K-AKT, JAK-STAT, PLCγ, and other signaling pathways. FGFR gene variations are widely present in lung cancer, liver cancer, intrahepatic cholangiocarcinoma, breast cancer, gastric cancer, uterine cancer, bladder cancer, and other solid tumors, but the type and frequency of FGFR mutations vary among different cancers.

FGFR has the following activity: (1) binding to FGF; (2) dimerizing FGFR due to the binding; (3) phosphorylating FGFR at specific tyrosine residues in FGFR due to the dimerization; (4) promoting the recruitment of adaptor proteins such as FGFR substrate 2α (FRS2α) due to the phosphorylation; and (5) transducing the signal produced by FGF stimulation to cells or tissues expressing FGFR or activating the signaling transduction.

The terms "FGFR2b" and "FGFR2IIIb" are used interchangeably to refer to the fibroblast growth factor receptor 2IIIb. An exemplary human FGFR2IIIb is shown in GenBank Accession No. NP_075259.4 (Jul. 7, 2013). The FGFR2IIIb protein generally binds to one or two or more FGFs selected from FGF1, FGF3, FGF7 (KGF), FGF10, FGF22, and FGF23. The FGFR2IIIb protein may bind to other FGFs, and may not bind to mutant forms of the listed FGFs.

The terms "FGFR2c" and "FGFR2IIIc" are used interchangeably to refer to the fibroblast growth factor receptor 2IIIc. An exemplary human FGFR2IIIc is shown in GenBank Accession No. NP_000132.3 (Jul. 7, 2013). The FGFR2IIIc protein generally binds to one or two or more FGFs selected from FGF1, FGF2, FGF4, FGF6, FGF9, FGF17, FGF18, FGF21, and FGF23. The FGFR2IIIc protein may bind to other FGFs, and may not bind to mutant forms of the listed FGFs.

In some embodiments, the antibody described herein binds to FGFR2b with a high affinity, and binds to FGFR2c with a low affinity or does not bind to FGFR2c. Generally, the antibody or the antigen-binding fragment thereof disclosed herein binds to FGFR2b with an affinity or binding activity of 10-fold, 100-fold, 1000-fold, or 10000-fold greater than that of FGFR2c.

The term "antibody-drug conjugate" (ADC) means that an antibody or an antigen-binding fragment thereof is linked to a biologically active drug via a stable linker unit. The linkage may be achieved by a covalent bond or a non-covalent interaction such as electrostatic force. To form an immunoconjugate, various linkers known in the art may be used.

The term "DAR" or "drug-to-antibody ratio" refers to the average number of small-molecule cytotoxic drugs linked per antibody molecule. In the antibody-drug conjugate of the present disclosure, the DAR is defined by the variable "n", which may be either an integer or a decimal.

The term "antigen-binding molecule" herein is used in its broadest sense and refers to a molecule that specifically binds to an antigen. Illustratively, the antigen-binding molecule includes, but is not limited to, an antibody or an antibody mimetic. The "antibody mimetic" refers to an organic compound or a binding domain that is capable of specifically binding to an antigen, but is not structurally related to an antibody. Illustratively, the antibody mimetic includes, but is not limited to, an affibody, an affitin, an affilin, a designed ankyrin repeat protein (DARPin), a nucleic acid aptamer, or a Kunitz domain peptide.

The term "antibody" herein is used in its broadest sense and refers to a polypeptide or a combination of polypeptides that comprises sufficient sequence from an immunoglobulin heavy chain variable region and/or sufficient sequence from an immunoglobulin light chain variable region for specifically binding to an antigen. The "antibody" used herein encompasses various forms and various structures as long as they exhibit the desired antigen-binding activity. The "antibody" used herein includes alternative protein scaffolds or artificial scaffolds having grafted complementarity determining regions (CDRs) or CDR derivatives. Such scaffolds include antibody-derived scaffolds comprising mutations introduced to, for example, stabilize the three-dimensional structure of the antibody, and fully synthetic scaffolds comprising, for example, biocompatible polymers. Such scaffolds may also include non-antibody derived scaffolds, such as scaffold proteins known in the art to be useful for grafting CDRs, including, but not limited to, tenascin, fibronectin, peptide aptamers, and the like.

The term "antibody" used herein includes intact antibodies and any antigen-binding fragments (i.e., "antigen-binding portion") or single chains thereof. The "antibody" includes a glycoprotein or an antigen-binding moiety thereof having at least two heavy (H) chains and two light (L) chains that are linked together by disulfide bonds. Each heavy chain consists of a heavy chain variable region (abbreviated herein as VH) and a heavy chain constant region. The heavy chain constant region consists of three domains: CH1, CH2, and CH3. Each light chain consists of a light chain variable region (abbreviated herein as VL) and a light chain constant region. The light chain constant region consists of one domain CL. The VH region and the VL region can be further subdivided into hypervariable regions called complementarity determining regions (CDRs), scattered among regions that are more conserved, termed framework regions (FRs). Each VH and VL consists of three CDRs and four FRs, which are arranged from the N-terminus to the C-terminus in the following order: FR1, CDR1, FR2, CDR2, FR3, CDR3, and FR4. The variable regions of the heavy and light chains comprise binding domains that can interact with antigens. The constant regions of the antibody may mediate the binding of the immunoglobulin to a host tissue or factor, and the host tissue or factor includes various cells (e.g., effector cells) of the immune system and the first component (C1q) of the classical complement system. The heavy chain constant regions of immunoglobulins differ in their amino acid composition and arrangement, and thus in their antigenicity. Accordingly, the "immunoglobulin" herein can be divided into five classes, or isoforms of immunoglobulins, i.e., IgM, IgD, IgG, IgA, and IgE, with their corresponding heavy chains being µ, δ, γ, α, and ε chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. The light chains can be divided into κ or λ chains according to the differences in the constant regions. Each of the five classes of Ig may have a κ chain or λ chain.

The term "antibody" herein also includes antibodies that do not comprise a light chain, e.g., heavy-chain antibodies (HCAbs) produced by Camelidae species, *Camelus dromedarius, Camelus bactrianus, Lama glama, Lama guanicoe, Vicugna pacos,* and the like, as well as immunoglobulin new antigen receptors (Ig new antigen receptor, IgNAR) found in *Chondrichthyes,* e.g., shark.

The term "antibody" used herein may be derived from any animal, including, but not limited to, human and non-human animals selected from primates, mammals, rodents, and vertebrates, such as Camelidae species, *Lama glama, Lama guanicoe, Vicugna pacos,* sheep, rabbits, mice, rats, or *Chondrichthyes* (e.g., shark).

The "antibody" herein includes, but is not limited to, monoclonal antibodies, polyclonal antibodies, monospecific antibodies, multispecific antibodies (e.g., bispecific antibodies), monovalent antibodies, multivalent antibodies, intact antibodies, fragments of an intact antibody, naked antibodies, conjugated antibodies, chimeric antibodies, humanized antibodies, or fully human antibodies.

The term "antibody" refers to an antibody obtained from a population of substantially homogeneous antibodies, that is, the individual antibodies constituting the population are identical and/or bind to the same epitope, except for possible variants (e.g., containing naturally occurring mutations or arising during the production of the formulation, such variants typically being present in minor amounts). In contrast to polyclonal antibody formulations that generally comprise different antibodies directed against different determinants (epitopes), each monoclonal antibody in a monoclonal antibody formulation is directed against a single determinant on the antigen. The modifier "monoclonal" herein shall not be construed as requiring the antibody or the antigen-binding molecule to be produced by any particular method. For example, monoclonal antibodies can be prepared by a variety of techniques, including (but not limited to) a hybridoma technique, a recombinant DNA method, a phage library display technique, methods that utilize transgenic animals containing all or part of human immunoglobulin loci, and other methods known in the art.

The term "antibody" refers to an antibody that is made and paired by the immune system of a multicellular organism. The term "engineered antibody" herein refers to a non-natural antibody obtained by a technique such as genetic engineering and antibody engineering. Illustratively, the "engineered antibody" includes humanized antibodies, small-molecule antibodies (e.g., scFv and the like), bispecific antibodies, and the like.

The term "monospecific" means having one or more binding sites, each of which binds to the same epitope of the same antigen.

The term "multispecific antibody" means having at least two antigen-binding sites, each of which binds to a different epitope of the same antigen or a different epitope of a different antigen. Thus, the terms such as "bispecific", "trispecific", and "tetraspecific" refer to the number of different epitopes to which an antibody/antigen-binding molecule can bind.

The term "valent" refers to the presence of a specified number of binding sites in an antibody/antigen-binding molecule. Thus, the terms "monovalent", "divalent", "tetravalent", and "hexavalent" refer to the presence of one binding site, two binding sites, four binding sites, and six binding sites, respectively, in an antibody/antigen-binding molecule.

The "full-length antibody", "complete antibody", and "intact antibody" are used interchangeably herein and refer to an antibody having a structure substantially similar to that of a natural antibody.

The "antigen-binding fragment" and "antibody fragment" are used interchangeably herein and do not have the entire structure of an intact antibody, but comprise only a portion of the intact antibody or a variant of the portion having the ability to bind to an antigen. Illustratively, the "antigen-binding fragment" or "antibody fragment" herein includes, but is not limited to, a Fab, an F(ab')₂, a Fab', a Fab'-SH, an Fd, an Fv, an scFv, a diabody, and a single domain antibody.

The term "single domain antibody" (sdAb) herein refers to a single domain antibody consisting of only one heavy chain variable region constructed by cloning a variable region of a heavy chain antibody, which is the smallest antigen-binding fragment having the complete functionality. Generally, a single domain antibody consisting of only one heavy chain variable region is constructed by obtaining a heavy chain antibody naturally lacking a light chain and a heavy chain constant region 1 (CH1) and then cloning a variable region of an antibody heavy chain.

The digestion of an intact antibody by papain produces two identical antigen-binding fragments, known as "Fab" fragments, each of which contains a heavy chain variable domain and a light chain variable domain, as well as a light chain constant domain and the first heavy chain constant domain (CH1). Thus, the term "Fab fragment" herein refers to an antibody fragment comprising a light chain fragment comprising the VL domain and the constant domain (CL) of a light chain, and the VH domain and the first constant domain (CH1) of a heavy chain. A Fab' fragment differs from the Fab fragment by the addition of a few residues (including one or more cysteines from an antibody hinge region) at the carboxyl terminus of the heavy chain CH1 domain. The Fab'-SH is a Fab' fragment in which the cysteine residue in the constant domain carries a free sulphydryl group. The pepsin treatment produces a F(ab')₂ fragment having two antigen-binding sites (two Fab fragments) and a portion of the Fc region.

The "Fv fragment" is the smallest fragment produced by IgG and IgM, comprising the intact antigen-binding site. The Fv fragment has the same binding properties and similar three-dimensional binding properties as Fab. The VH and VL chains of the Fv fragment are bound together by a non-covalent interaction.

The term "scFv (single-chain variable fragment)" refers to a single polypeptide chain comprising VL and VH domains, wherein the VL and VH are linked via a linker. Such scFv molecules may have a general structure: NH₂-VL-linker-VH-COOH or NH₂-VH-linker-VL-COOH. An appropriate linker in the prior art consists of GGGGS amino acid sequence repeats or a variant thereof. For example, a linker having the amino acid sequence (GGGGS)₄ (SEQ ID NO. 37) may be used, but a variant thereof may also be used. In some cases, there may also be disulfide bonds between the VH and VL of the scFv, forming a disulfide-linked Fv (dsFv).

The term "diabody" refers to an antibody having VH and VL domains expressed on a single polypeptide chain, but using a linker that is too short to allow pairing between the two domains on the same chain, thereby forcing the domains to pair with complementary domains of another chain and producing two antigen-binding sites.

The term "chimeric antibody" refers to an antibody in which a portion of the light or/and heavy chain is derived from one antibody (which may be derived from a particular species or belong to a particular antibody class or subclass) and another portion of the light or/and heavy chain is derived from another antibody (which may be derived from the same or a different species or belong to the same or a different antibody class or subclass), but which nevertheless retains binding activity to a target antigen. For example, the term "chimeric antibody" may include an antibody (e.g., a human-murine chimeric antibody) in which the heavy and light chain variable regions of the antibody are derived from a first antibody (e.g., a murine antibody) and the heavy and light chain constant regions of the antibody are derived from a second antibody (e.g., a human antibody).

The term "humanized antibody" refers to a genetically engineered non-human antibody that has an amino acid sequence modified to increase the homology to the sequence of a human antibody. Generally, all or part of the CDRs of a humanized antibody are derived from a non-human antibody (donor antibody), and all or part of the non-CDR regions (e.g., variable region FRs and/or constant regions) are derived from a human immunoglobulin (receptor antibody). The humanized antibody generally retains or partially retains the desired properties of the donor antibody, including, but not limited to, antigen specificity, affinity, reactivity, the ability to increase the activity of immune cells, the ability to enhance immune response, and the like.

The term "fully human antibody" refers to an antibody having variable regions in which both the FRs and CDRs are derived from human germline immunoglobulin sequences. Furthermore, if the antibody comprises constant regions, the constant regions are also derived from human germline immunoglobulin sequences. The fully human antibody herein may include amino acid residues that are not encoded by human germline immunoglobulin sequences (e.g., mutations introduced by random or site-specific mutagenesis *in vitro* or by somatic mutations *in vivo).* However, the "fully human antibody" herein does not include antibodies in which CDR sequences derived from the germline of another mammalian species (e.g., mouse) have been grafted onto human framework sequences.

The term "naked antibody" herein refers to an antibody that is not linked, fused, or conjugated to another agent or molecule (e.g., a label or drug), peptide, or polypeptide. In specific embodiments, the naked antibody expressed by mammalian host cells can be glycosylated by the glycosylation machinery (e.g., glycosylases) of the host cells. In certain embodiments, the naked antibody is not glycosylated when expressed by a host cell that does not have its own glycosylation machinery (e.g., a glycosylase). In certain embodiments, the naked antibody is an intact antibody, while in other embodiments, the naked antibody is an antigen-binding fragment of an intact antibody, such as a Fab antibody.

The term "variable region" refers to a region of a heavy or light chain of an antibody involved in the binding of the antibody to an antigen. The "heavy chain variable region" is used interchangeably with "VH" and "HCVR", and the "light chain variable region" is used interchangeably with "VL" and "LCVR". The heavy and light chain variable domains (VH and VL, respectively) of natural antibodies generally have similar structures, each of which contains four conserved framework regions (FRs) and three hypervariable regions (HVRs). A single VH or VL domain may be sufficient to provide the antigen-binding specificity. The terms "complementarity determining region" and "CDR" herein are used interchangeably and generally refer to a hypervariable region (HVR) of a heavy chain variable region (VH) or a light chain variable region (VL), which is also known as the complementarity determining region as it is precisely complementary to an antigenic epitope in spatial structure, wherein the heavy chain variable region CDR may be abbreviated as HCDR and the light chain variable region CDR may be abbreviated as LCDR. The terms "framework region" and "FR" are used interchangeably and refer to those amino acid residues of an antibody heavy chain variable region or light chain variable region other than CDRs. Generally, a typical antibody variable region consists of 4 FRs and 3 CDRs in the following order: FR1-CDR1-FR2-CDR2-FR3-CDR3-FR4.

The "CDR" used herein may be labeled and defined in a manner well known in the art, including, but not limited to, Kabat numbering scheme, Chothia numbering scheme, or IMGT numbering scheme; the tool sites used include, but are not limited to, AbRSA site (http://cao.labshare.cn/AbRSA/cdrs.php), abYsis site (www.abysis.org/abysis/sequence_input/key_annotation/key_annotation.cgi), and IMGT site (http://www.imgt.org/3Dstructure-DB/cgi/DomainGapAlign.cgi#results). The CDR used herein includes overlaps and subsets of amino acid residues defined in different ways.

The term "heavy chain constant region" used herein refers to the carboxyl-terminal portion of an antibody heavy chain that is not directly involved in the binding of the antibody to an antigen, but exhibits effector functions, such as interaction with an Fc receptor, which has a more conserved amino acid sequence relative to the variable domain of the antibody. The "heavy chain constant region" is selected from: a CH1 domain, a hinge region, a CH2 domain, a CH3 domain, or a variant or fragment thereof. The "heavy chain constant region" includes a "full-length heavy chain constant region" having a structure substantially similar to that of a natural antibody constant region and a "heavy chain constant region fragment" including only "a portion of the full-length heavy chain constant region". Illustratively, a typical "full-length antibody heavy chain constant region" consists of the CH1 domain-hinge region-CH2 domain-CH3 domain. When the antibody is IgE, it further comprises a CH4 domain; and when the antibody is a heavy chain antibody, it does not comprise a CH1 domain. Illustratively, a typical "heavy chain constant region fragment" is selected from an Fc domain or a CH3 domain.

The term "light chain constant region" herein refers to the carboxyl-terminal portion of an antibody light chain that is not directly involved in the binding of the antibody to an antigen. The light chain constant region is selected from a constant κ domain or a constant λ domain.

The term "Fc" herein refers to the carboxyl-terminal portion of an antibody that is formed by the hydrolysis of an intact antibody by papain, which typically comprises the CH3 and CH2 domains of the antibody. The Fc region includes, for example, an Fc region of native sequences, a recombinant Fc region, and a variant Fc region. Although the boundaries of the Fc region of an immunoglobulin heavy chain may vary slightly, the human IgG heavy chain Fc region is generally defined to extend from an amino acid residue at position Cys226, or from Pro230, to the carboxyl terminus thereof. The C-terminal lysine of the Fc region (residue 447 according to the Kabat numbering scheme) may be removed, for example, during production or purification of the antibody, or by recombinant engineering of the nucleic acid encoding the heavy chain of the antibody, and thus, the Fc region may or may not include Lys447.

The term "conservative amino acid" herein generally refers to amino acids that belong to the same class or have similar characteristics (e.g., charge, side chain size, hydrophobicity, hydrophilicity, backbone conformation, and rigidity). Illustratively, the amino acids in each of the following groups are conservative amino acid residues of each other, and the substitutions by amino acid residues within the groups are conservative amino acid substitutions:
1) alanine (A), serine (S), and threonine (T);
2) aspartic acid (D) and glutamic acid (E);
3) asparagine (N) and glutamine (Q);
4) arginine (R), lysine (K), and histidine (H);
5) isoleucine (I), leucine (L), methionine (M), and valine (V); and
6) phenylalanine (F), tyrosine (Y), and tryptophan (W).

The term "identity" herein can be obtained by calculating as follows: to determine the percent "identity" of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison purpose). Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

The term "nucleic acid" herein includes any compound and/or substance that comprises a polymer of nucleotides. Each nucleotide consists of a base, in particular a purine or pyrimidine base (i.e., cytosine (C), guanine (G), adenine (A), thymine (T), or uracil (U)), a saccharide (i.e., deoxyribose or ribose), and a phosphate group. Generally, a nucleic acid molecule is described as a sequence of bases, whereby the bases represent the primary structure (linear structure) of the nucleic acid molecule. The sequence of bases is generally expressed as 5' to 3'. In this context, the term "nucleic acid molecule" encompasses deoxyribonucleic acid (DNA), including, e.g., complementary DNA (cDNA) and genomic DNA; ribonucleic acid (RNA), in particular messenger RNA (mRNA); synthetic forms of DNA or RNA; and polymers comprising a mixture of two or more of these molecules. The nucleic acid molecule may be linear or cyclic. Furthermore, the term "nucleic acid molecule" includes both sense and antisense strands, as well as single- and double-stranded forms. Moreover, the nucleic acid molecules described herein may contain natural or non-natural nucleotides. Examples of non-natural nucleotides include modified nucleotide bases having derived sugar or phosphate backbone linkages or chemically modified residues. The nucleic acid molecule also encompasses DNA and RNA molecules suitable for use as vectors for direct expression of the antibodies of the present disclosure *in vitro* and/or *in vivo,* e.g., in a host or patient. Such DNA (e.g., cDNA) or RNA (e.g., mRNA) vectors may be unmodified or modified. For example, mRNA can be chemically modified to enhance the stability of the RNA vector and/or the expression of the encoded molecule such that the mRNA can be injected into a subject to produce antibodies *in vivo* (see, e.g., EP 2 101 823 B1, which is incorporated herein by reference).

As used herein, the term "vector" includes nucleic acid vectors, e.g., DNA vectors (e.g., plasmids), RNA vectors, viruses, or other suitable replicons (e.g., viral vectors). Various vectors have been developed for the delivery of polynucleotides encoding foreign proteins into prokaryotic or eukaryotic cells. The expression vector of the present disclosure contains polynucleotide sequences as well as additional sequence elements, e.g., for expressing proteins and/or integrating these polynucleotide sequences into the genome of mammalian cells. Some vectors that may be used to express the antibody and antibody fragment of the present disclosure include plasmids comprising regulatory sequences (e.g., promoter and enhancer regions) that direct gene transcription. Other useful vectors for expressing the antibody and antibody fragment contain polynucleotide sequences that enhance the rate of translation of these genes or improve the stability or nuclear export of mRNA produced by gene transcription. These sequence elements include, for example, 5' and 3' untranslated regions, internal ribosome entry sites (IRESs), and polyadenylation signal sites, so as to direct the effective transcription of the gene carried on the expression vector. The expression vector of the present disclosure may also contain a polynucleotide encoding a marker for selecting cells comprising such a vector. Examples of suitable markers include genes encoding resistance to antibiotics (e.g., ampicillin, chloramphenicol, kanamycin, or nourseothricin).

The term "host cell" used herein refers to a cell into which an exogenous nucleic acid has been introduced, including the progeny of such a cell. Host cells include "transformants" and "transformed cells", which include primary transformed cells and progenies derived therefrom, regardless of the number of passages. Progenies may not be exactly the same as the parent cells in terms of nucleic acid content, and may contain mutations. Mutant progenies having the same functionality or biological activity that are screened or selected from the primary transformed cells are included herein. The percent identity between two sequences varies with the identical positions shared by the sequences, taking into account the number and length of gaps to be introduced for optimal alignment of the two sequences.

The "n is a real number of 1 to 16" herein means that n is any real number greater than or equal to 1 and less than or equal to 16.

Herein, " 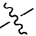 " denotes a linking site.

The illustration method for the racemic or enantiomerically pure compounds herein is from Maehr, J. Chem. Ed. 1985, 62:114-120, which is incorporated herein by reference. Unless otherwise stated, the absolute configuration of a stereogenic center is represented by a wedged bond and a wedged dashed bond ( 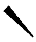 and 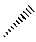 ), and the relative configuration of a stereogenic center (e.g., *cis-* or *trans-*configuration of alicyclic compounds) is represented by a black solid bond and a dashed bond ( 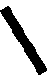 and 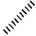 ).

The term "stereoisomer" refers to isomers resulting from different spatial arrangements of atoms in a molecule, including cis-trans isomers, enantiomers, and diastereoisomers.

The compound of the present disclosure may have an asymmetric atom such as a carbon atom, a sulfur atom, a nitrogen atom, and a phosphorus atom, or an asymmetric double bond, and thus the compound of the present disclosure may be present in the form of a particular geometric isomer or stereoisomer. The form of a particular geometric isomer or stereoisomer may be cis and trans isomers, E and Z geometric isomers, (-)- and (+)-enantiomers, (R)- and (S)-enantiomers, diastereoisomers, (D)-isomers, (L)-isomers, and racemic mixtures or other mixtures thereof, such as an enantiomer or diastereoisomer enriched mixture, and all of the above isomers, as well as mixtures thereof, fall within the definition scope of the compound of the present disclosure. An additional asymmetric carbon atom, asymmetric sulfur atom, asymmetric nitrogen atom, or asymmetric phosphorus atom may be present in substituents such as alkyl. All of these isomers and mixtures thereof involved in the substituents are also encompassed within the definition scope of the compound of the present disclosure. The compound containing an asymmetric atom of the present disclosure can be separated in an optically active pure form or in a racemic form. The optically active pure form can be obtained by resolving a racemic mixture or by synthesis using chiral starting materials or chiral reagents.

The term "substituted" means that any one or more hydrogen atoms on a specific atom are replaced by substituents, as long as the valence of the specific atom is normal and the compound resulting from the substitution is stable. When the substituent is oxo (i.e., =O), it means that two hydrogen atoms are replaced, and oxo will not be present on an aromatic group.

The term "optional" or "optionally" means that the subsequently described event or circumstance may or may not occur. The description includes instances where the event or circumstance occurs and instances where the event or circumstance does not occur. For example, ethyl being "optionally" substituted with halogen means that the ethyl may be unsubstituted (CH₂CH₃), monosubstituted (CH₂CH₂F, CH₂CH₂Cl, or the like), polysubstituted (CHFCH₂F, CH₂CHF₂, CHFCH₂Cl, CH₂CHCl₂, or the like), or fully substituted (CF₂CF₃, CF₂CCl₃, CCl₂CCl₃, or the like). Those skilled in the art will appreciate that for any group containing one or more substituents, no substitution or substituting pattern that is spatially impossible and/or cannot be synthesized will be introduced.

The Cₘ-Cₙ herein means having an integer number of carbon atoms within the range of m to n.

The term "alkyl" refers to a hydrocarbyl group with a general formula of CₙH₂ₙ₊₁. The alkyl may be linear or branched. The term "C₁-C₆ alkyl" should be understood to represent a linear or branched saturated hydrocarbon group having 1, 2, 3, 4, 5, or 6 carbon atoms. The alkyl includes, but is not limited to, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, and the like. The term "C₁-C₃ alkyl" refers to an alkyl group containing 1 to 3 carbon atoms, such as methyl, ethyl, n-propyl, or isopropyl.

The "C₁-C₆ alkyl" described herein may further include "C₁-C₃ alkyl".

The term "cycloalkyl" refers to a fully saturated carbon ring that is present in the form of a monocyclic ring, a fused ring, a bridged ring, a spiro ring, or the like. The term "C₃-C₆ cycloalkyl" should be understood to represent a saturated monocyclic, fused, spiro, or bridged ring having 3-6 carbon atoms. Specific examples include, but are not limited to, cyclopropyl, cyclobutyl, cyclopentyl, cyclohexyl, and the like.

The term "heterocyclyl" refers to a fully saturated or partially saturated monocyclic, fused cyclic, spiro cyclic, or bridged cyclic group, and ring atoms of the group include 1-5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), -S(=O)₂-, -S(=O)-, -P(=O)₂-, -P(=O)-, -NH-, -S(=O)(=NH)-, -C(=O)NH-, -NHC(=O)NH-, and the like. The term "4- to 7-membered heterocyclyl" refers to a heterocyclyl group having 4, 5, 6, or 7 ring atoms, wherein 1-3 ring atoms are independently selected from the heteroatoms or heteroatom groups described above. The term "5- to 6-membered heterocyclyl" refers to a heterocyclyl group having 5 or 6 ring atoms, wherein 1-3 ring atoms are independently selected from the heteroatoms or heteroatom groups described above. Examples of 4-membered heterocyclyl include, but are not limited to, azetidinyl or oxetanyl; examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, or 2,5-dihydro-1*H*-pyrrolyl; examples of 6-membered heterocyclyl include, but are not limited to, tetrahydropyranyl, piperidinyl, morpholinyl, dithianyl, thiomorpholinyl, piperazinyl, trithianyl, tetrahydropyridinyl, or 4*H-*[1,3,4]thiadiazinyl; examples of 7-membered heterocyclyl include, but are not limited to, diazepanyl. The "4- to 7-membered heterocyclyl" may include the ranges of "4- to 7-membered heterocycloalkyl", "5- to 6-membered heterocyclyl", "5- to 6-membered heterocycloalkyl", and the like.

The term "halo-" or "halogen" refers to fluorine, chlorine, bromine, and iodine.

The term "treatment" refers to surgical or therapeutic treatment for the purpose of preventing or slowing (reducing) the progression of an undesired physiological or pathological change, e.g., cancer, an autoimmune disease, and virus infection, in a subject being treated. Beneficial or desired clinical results include, but are not limited to, the alleviation of symptoms, the decrease in the severity of disease, the stabilization (i.e., not worsening) of the state of disease, the delay or slowing of disease progression, the amelioration or palliation of the state of disease, and remission (whether partial or total), whether detectable or undetectable. Subjects in need of treatment include those already having a disorder or disease, as well as those who are susceptible to a disorder or disease or those who intend to prevent a disorder or disease. When referring to terms such as slowing, alleviation, decrease, palliation, and remission, elimination, disappearance, nonoccurrence, and the like are also included. The term "effective amount" refers to an amount of a therapeutic agent that, when administered alone or in combination with another therapeutic agent to a cell, tissue, or subject, is effective to prevent or alleviate symptoms of a disease or the progression of the disease. The "effective amount" also refers to an amount of a compound that is sufficient to alleviate a symptom, e.g., to treat, cure, prevent, or alleviate a related medical disorder, or to increase the rates at which such a disorder is treated, cured, prevented, or alleviated. When the active ingredient is administered alone to an individual, the therapeutically effective dose refers to the amount of the ingredient alone. When a combination is used, the therapeutically effective dose refers to the combined amounts of the active ingredients that produce the therapeutic effect, whether administered in combination, sequentially, or simultaneously. The term "subject" refers to an organism that receives treatment for a particular disease or disorder as described in the present disclosure. Examples of subjects and patients include mammals, such as humans, primates (e.g., monkeys), or non-primate mammals, that receive treatment for a disease or disorder.

The amount of the compound of the present disclosure constituting the "therapeutically effective amount" varies depending on the compound, the state of disease and its severity, the mode of administration, and the age of the mammal to be treated, but can be determined routinely by those skilled in the art in accordance with their knowledge and the content of the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to salts of pharmaceutically acceptable acids or bases, including salts formed from the compound and an inorganic or organic acid, and salts formed from the compound and an inorganic or organic base.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, and water.

The word "comprise" and variations thereof such as "comprises" or "comprising" may be interpreted in an open and non-exclusive sense, i.e., "including but not limited to".

The present disclosure also includes isotopically labeled compounds of the present disclosure that are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present disclosure (e.g., those labeled with ³H and ¹⁴C) can be used to analyze compounds and/or substrate tissue distribution. Tritiation (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

The pharmaceutical composition of the present disclosure may be suitable for parenteral administration, such as a sterile solution, suspension, or lyophilized product in a suitable unit dosage form. For example, the pharmaceutical composition of the present disclosure may be in the form of a sterile injectable aqueous solution for intramuscular or subcutaneous administration. Other vehicles or solvents such as water, Ringer's solution, or isotonic sodium chloride solution are acceptable when the pharmaceutical composition of the present disclosure is used.

The pharmaceutical composition described herein may also comprise a therapeutic antibody or a chemotherapeutic agent, such as a PD-1/PD-L1 inhibitor. The pharmaceutical composition can be packaged as a combination in one pharmaceutical package, or the anti-FGFR2b antibody-drug conjugate and the additional therapeutic agent described herein are packaged separately and independently in different kits.

The compounds of the present disclosure can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions of the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

An important consideration in synthetic route planning in the art is the selection of suitable protecting groups for reactive functional groups (e.g., amino and carboxyl in the present disclosure). For example, reference may be made to Greene's Protective Groups in Organic Synthesis (4th Ed.) Hoboken, New Jersey: John Wiley & Sons, Inc. All references cited in the present disclosure are incorporated herein in their entirety.

The term "cancer" used herein refers to or describes a physiological condition in mammals that is typically characterized by unregulated cell growth. Included in this definition are benign and malignant cancers. The term "tumor" or "neoplasm" used herein refers to all neoplastic cell growth and proliferation, whether malignant or benign, and all pre-cancerous and cancerous cells and tissues. The terms "cancer" and "tumor" are not mutually exclusive when referred to herein.

Non-limiting examples of cancer include gastric cancer, squamous cell lung cancer, triple-negative breast cancer, ovarian cancer, endometrial carcinoma, pancreatic cancer, intrahepatic cholangiocarcinoma, colorectal cancer, and esophageal cancer. In some embodiments, the cancer comprises FGFR2 gene amplification. In some embodiments, the FGFR2 amplification comprises an FGFR2:CEN10 (chromosome 10 centromere) ratio of >3. In some embodiments, the cancer comprising the FGFR2 gene overexpresses FGFR2IIIb. In some embodiments, the cancer comprising the FGFR2 overexpression overexpresses FGFR2IIIb to a greater extent than FGFR2IIIc. In some embodiments, the cancer comprising the FGFR2 amplification expresses FGFR2IIIb at a normalized level that is 2-fold, 3-fold, 5-fold, or 10-fold greater than the normalized level of FGFR2IIIc expression. In some embodiments, the expression level is normalized to GUSB. In some embodiments, the cancer overexpresses FGFR2IIIb but does not comprise FGFR2 gene amplification. In some embodiments, the gastric cancer comprises an FGFR2 gene amplification. In some embodiments, the gastric cancer comprising the FGFR2 gene amplification overexpresses FGFR2IIIb. In some embodiments, the gastric cancer comprising the FGFR2 gene amplification overexpresses FGFR2IIIb to a greater extent than FGFR2IIIc. In some embodiments, the gastric cancer comprising the FGFR2 gene amplification expresses FGFR2IIIb at a normalized level that is 2-fold, 3-fold, 5-fold, or 10-fold greater than the normalized level of FGFR2IIIc expression. In some embodiments, the expression level is normalized to GUSB. In some embodiments, the gastric cancer overexpresses FGFR2IIIb but does not comprise FGFR2 gene amplification. In some embodiments, the overexpression is mRNA overexpression. In some embodiments, the overexpression is protein overexpression.

The term "EC50" used herein refers to the half maximum effective concentration, which includes the antibody concentration that induces a halfway response between the baseline and maximum after a specified exposure time. EC50 essentially represents the antibody concentration at which 50% of the maximal effect is observed, and can be measured by methods known in the art.

### Examples

### Example 1: Preparation of anti-human FGFR2b antibodies and reference antibodies

### 1.1 Preparation of anti-human FGFR2b monoclonal antibodies

Three anti-human FGFR2b monoclonal antibodies Ab-1, Ab-2, and Ab-3 were obtained in-house by research. The nucleic acid sequences encoding the heavy chain variable regions (VHs) and light chain variable regions (VLs) of the antibodies were recombined into expression vector pTT5 carrying a signal peptide and the sequences of the heavy chain constant region (CH) and light chain constant region (CL) of human antibody IgG1, respectively, so as to give recombinant plasmids expressing VH-CH and VL-CL. **Table 1** shows the VH and VL sequences of the antibodies, signal peptide sequence, and CH and CL sequences of human antibody IgG1, and **Table 2** shows the Kabat analysis results of the CDR sequences.

**Table 1. Sequences of variable regions, signal peptide, and constant regions of anti-FGFR2b monoclonal antibodies**

| **Sequence name** | SEQ ID NO. | **Amino acid sequence** |
|---|---|---|
| Ab-1 VH | 1 | EVQLVQSGAEVKKPGASVKVSCKASGYIFTSYWMHWVRQAPGQRLE WMGAIYPGNSDTNYNQKFKGRVTITADASASTAYMELSSLRSEDTAV YYCIRPFAYWGQGTLVTVSS |
| Ab-1 VL | 2 | DIVMTQTPLSLPVTPGEPASISCKASQSVGNNVAWYLQKPGQSPKLLI YYASNRYTGVPDRFSGSGSGTDFTLKISRVEAEDVGVYYCQQYYSSP YTFGQGTKLEIK |
| Ab-2 VH | 3 | EVQLVQSGAEVKKPGSSVKVSCKTSGYAFSSYWMNWVRQAPGQGLE WMGQIYPENADTNYQGKFEGRVTITADKSTSTAYMELSSLRSEDTAV YYCANPYYGSAMDYWGQGTTVTVSS |
| Ab-2 VL | 4 | DIVMTQTPLSLPVTPGEPASISCKSSQSLLYSSSQKNFLAWYLQKPGQS PKLLIYWASTRESGVPDRFSGSGSGTDFTLKISRVEAEDVGVYYCQQY YSNPITFGQGTKLEIK |
| Ab-3 VH | 5 | EVQLVQSGAEVKKPGSSVKVSCKASGYTFPSYNMHWVRQAPGQGLE WMGTIYPDNADSSYNQKFKGRVTITADKSTSTAYMELSSLRSEDTAVY YCARGGFDSWGQGTTVTVSS |
| Ab-3 VL | 6 | DIQMTQSPSSLSASVGDRVTITCKASQDVNNNVAWYQQKPGKAPKLL IYSASYRYTGVPSRFSGSGSGTDFTFTISSLQPEDIATYYCQQHYITPYT FGGGTKVEIK |
| Human IgG1 CH | 7 | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTV LHQDWLNGKEYKCKVSNKALPAPIEKTISKAKGQPREPQVYTLPPSRE EMTKNQVSLTCLVKGFYPSDIAVEWESNGQPENNYKTTPPVLDSDGSF FLYSKLTVDKSRWQQGNVFSCSVMHEALHNHYTQKSLSLSPGK |
| Human IgG1 CL | 8 | |

**Table 2. Kabat analysis of CDR sequences of anti-FGFR2b monoclonal antibodies**

| **Sequence name** | **CDR1** | **SEQ ID NO.** | **CDR2** | **SEQ ID NO.** | **CDR3** | **SEQ ID NO.** |
|---|---|---|---|---|---|---|
| Ab-1 VH | SYWMH | 9 | AIYPGNSDTNYNQK FKG | 10 | PFAY | 11 |
| Ab-1 VL | KASQSVGNNVA | 12 | YASNRYT | 13 | QQYYSSP YT | 14 |
| Ab-2 VH | SYWMN | 15 | QIYPENADTNYQGK FEG | 16 | PYYGSAM DY | 17 |
| Ab-2 VL | KSSQSLLYSSSQ KNFLA | 18 | WASTRES | 19 | QQYYSNP IT | 20 |
| Ab-3 VH | SYNMH | 21 | TIYPDNADSSYNQK FKG | 22 | GGFDS | 23 |
| Ab-3 VL | KASQDVNNNVA | 24 | SASYRYT | 25 | QQHYITP YT | 26 |

The expression plasmid and the transfection reagent PEI (purchased from Polysciences, Cat. No. 24765-1) were added to OPTI-MEM (purchased from Gibco, Cat. No. 11058021). The mixture was well mixed and let stand for 15 min. Expi293F cells (purchased from Thermofisher, Cat. No. A14527) were added, and the system was incubated on a shaker at 37 °C in 5% CO₂ at 120 rpm. On day 2 of the transfection, OPM-293 ProFeed (purchased from Shanghai OPM Biosciences Co., Ltd., Cat. No. F081918-001) and 6 g/L glucose (purchased from Sigma-Aldrich, Cat. No. G8270-5KG) were added.

On day 6 of the transfection, the cell culture supernatant was collected.

The cell culture supernatant was purified on a Protein A column (purchased from Cytiva, Cat. No. 17549802) to separate the antibodies. The Protein A column was equilibrated with 3-5 column volumes of an equilibration buffer (PBS, pH 7.4; purchased from Sangon Biotech, Cat. No. B548117-0500), and the clarified culture supernatant was loaded on the Protein A column at a controlled flow rate of 10 mL/min. After the sample loading, the Protein A column was washed with 3-5 column volumes of the equilibration buffer. The proteins bound to the Protein A column were eluted with an eluent (50 mM NaAc-HAc, pH 3.5). The eluted proteins were collected and adjusted to a neutral pH with Tris buffer (purchased from Sinopharm, Cat. No. 30188336). The samples were appropriately concentrated and further purified by gel chromatography Superdex200 (purchased from Cytiva, Cat. No. 28990946) pre-equilibrated with PBS. The aggregates were removed, and the monomer peaks were collected and filtered through a 0.22 µm filter (purchased from Millipore, Cat. No. SLGVR13SL) for sterilization. The concentration was measured by Nanodrop (purchased from Thermofisher), the antibody purity was determined using HPLC-SEC, and the endotoxin content in the antibody solution was measured using an endotoxin detection kit (purchased from A&C Biological). Qualified samples were aliquoted and preserved at -80 °C.

### 1.2 Preparation of reference antibodies

The antibody sequence of the anti-FGFR2b positive control bemarituzumab (FPA144) from Amgen is the HuGAL-FR21 sequence in Patent No. US8603987B2. The sequence was obtained according to the patent, as shown in **Table 3,** and the antibody was designated as FPA144-hIgG1. The antibody sequences of the anti-FGFR2b positive control antibodies mAb1 and mAb2 from Regeneron come from Patent No. WO2022087243A1. The sequences obtained according to the patent are shown in **Table 3.** The isotype control antibody was an antibody against fluorescein isothiocyanate (FITC) that does not bind to FGFR2. The sequence is shown in **Table 3,** and the antibody was designated as Isotype. Reference was made to the expression and purification methods for antibodies in 1.1 to obtain the reference antibodies.

**Table 3. Heavy and light chain sequences of reference antibodies**

| **Sequence name** | SEQ ID NO. | **Amino acid sequence** |
|---|---|---|
| FPA 144-hIgG1 heavy chain | 27 | QVQLVQSGAEVKKPGSSVKVSCKASGYIFTTYNVHWVRQAPGQ GLEWIGSIYPDNGDTSYNQNFKGRATITADKSTSTAYMELSSLRSE DTAVYYCARGDFAYWGQGTLVTVSSASTKGPSVFPLAPSSKSTSGG TAALGCLVKDYFPEPVTVSWNSGALTSGVHTFPAVLQSSGLYSLSSVVT VPSSSLGTQTYICNVNHKPSNTKVDKRVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKALP APIEKTISKAKGQPREPQVYTLPPSREEMTKNQVSLTCLVKGFYPSDIA VEWESNGQPENNYKTTPPVLDSDGSFFLYSKLTVDKSRWQQGNVFSC SVMHEALHNHYTQKSLSLSPGK |
| FPA 144-hIgG1 light chain | 28 | DIQMTQSPSSLSASVGDRVTITCKASQGVSNDVAWYQQKPGKAPK LLIYSASYRYTGVPSRFSGSGSGTDFTFTISSLQPEDIATYYCQQHS TTPYTFGQGTKLEIKRTVAAPSVFIFPPSDEQLKSGTASVVCLLNNFYP REAKVQWKVDNALQSGNSQESVTEQDSKDSTYSLSSTLTLSKADYEKH KVYACEVTHQGLSSPVTKSFNRGEC |
| mAb1 heavy chain | 29 | QVQLVESGGGVVQPGRSLRLSCAASGFTFSSYGMHWVRQAPGK GLEWVAVIWYDGSNKYYADSVKGRFTISRDNSKNTLYLQMNSLR AEDTAVYYCAREESSASGVDYWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFLGGPSVFLFPPKPKDTLMISRTPEVTCVVVDVSQEDPEVQFNWY VDGVEVHNAKTKPREEQFNSTYRVVSVLTVLHQDWLNGKEYKCKVSN KGLPSSIEKTISKAKGQPREPQVYTLPPSQEEMTKNQVSLTCLVKGFY PSDIAVEWESNGQPENNYKTTPPVLDSDGSFFLYSRLTVDKSRWQEG NVFSCSVMHEALHNHYTQKSLSLSLGK |
| mAb1 light chain | 30 | EIVLTQSPGTLSLSPGERATLSCRASQSVSTRYLAWYQQKRGQAPR LLIYGASSRATGIPDRFSGSGSGTDFTLTISRLEPEDFAVYYCQEYG SSSITFGQGTRLEIKRTVAAPSVFIFPPSDEQLKSGTASVVCLLNNFYP REAKVQWKVDNALQSGNSQESVTEQDSKDSTYSLSSTLTLSKADYEKH KVYACEVTHQGLSSPVTKSFNRGEC |
| mAb2 heavy chain | 31 | QVQLVESGGGVVQPGKSLRLSCAASGFTFSNYGMHWVRQTPVK GLEWVTLIWYDGSNKYYTDSVKGRFTISRDNSKNTLYLQMNSLR SEDTAVYYCAREGNWNYGHAFDIWGQGTMVTVSSASTKGPSVFP LAPCSRSTSESTAALGCLVKDYFPEPVTVSWNSGALTSGVHTFPAVLQS SGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCK VSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQ EGNVFSCSVMHEALHNHYTQKSLSLSLGK |
| mAb2 light chain | 32 | DIQMTQSPSTLSASVGDRVTITCRASQNIGNWLAWYQQKPGKAP NLLIYKASTLESGVPSRFSGSGSGTEFTLTISSLQPDDFATYNCQQY NSYSPTFGQGTKLEIKRTVAAPSVFIFPPSDEQLKSGTASVVCLLNNFY PREAKVQWKVDNALQSGNSQESVTEQDSKDSTYSLSSTLTLSKADYEK HKVYACEVTHQGLSSPVTKSFNRGEC |
| Isotype heavy chain | 33 | EVKLDETGGGLVQPGRPMKLSCVASGFTFSDYWMNWVRQSPEK GLEWVAQIRNKPYNYETYYSDSVKGRFTISRDDSKSSVYLQMNN LRVEDMGIYYCTGSYYGMDYWGQGTSVTVSSASTKGPSVFPLAPS SKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFN WYVDGVEVHNAKTKPREEQYNSTYRVVSVLTVLHQDWLNGKEYKCK VSNKALPAPIEKTISKAKGQPREPQVYTLPPSREEMTKNQVSLTCLVKG FYPSDIAVEWESNGQPENNYKTTPPVLDSDGSFFLYSKLTVDKSRWQQ GNVFSCSVMHEALHNHYTQKSLSLSPGK |
| Isotype light chain | 34 | DVVMTQTPLSLPVSLGDQASISCRSSQSLVHSNGNTYLRWYLQKP GQSPKVLIYKVSNRFSGVPDRFSGSGSGTDFTLKISRVEAEDLGVY FCSQSTHVPWTFGGGTKLEIKRTVAAPSVFIFPPSDEQLKSGTASVVC LLNNFYPREAKVQWKVDNALQSGNSQESVTEQDSKDSTYSLSSTLTLS KADYEKHKVYACEVTHQGLSSPVTKSFNRGEC |

| | | |
|---|---|---|
| Note: antibody variable region + constant region (italic) | | |

The affinity of the antibodies to human FGFR2(β)b protein was determined by surface plasmon resonance (SPR). The antibodies were captured on a Protein A chip (purchased from Cytiva, Cat. No. 29-127-558). The sample and running buffer was HBS-EP+ (10 mM HEPES, 150 mM NaCl, 3 mM EDTA, 0.05% surfactant P20; purchased from Cytiva, Cat. No. BR-1006-69). The flow cell was set at 25 °C. The sample block was set at 16 °C. Both were pretreated with the running buffer. In each cycle, the antibodies were captured on the Protein A chip, and human FGFR2(β)b-His antigen protein at a single concentration was injected. The association and dissociation processes of the antibodies with the antigen protein were recorded, and finally, the chip was regenerated in glycine, pH 1.5 (Cytiva; BR-1003-54). The association was determined by maintaining different concentrations of human FGFR2(β)b-His protein in the injections for 240 s, wherein the flow rate was 30 µL/min, and the protein was serially 1:1 diluted from 200 nM to give a total of 5 concentrations. The dissociation phase was monitored for up to 600 s and triggered by switching from the sample solution to the running buffer. The surface was regenerated by washing with 10 mM glycine solution (pH 1.5) at a flow rate of 30 µL/min for 30 s. The difference in bulk refractive index was corrected by subtracting the responses obtained from the reference channel. Blank injections were also subtracted (= double reference). To calculate the apparent KD and other kinetic parameters, the Langmuir 1:1 model was used. The association rate (ka), dissociation rate (kd), and binding affinity (KD) of antibodies for human FGFR2(β)b-His protein are shown in **Table 4.** All the three candidate humanized antibodies exhibited good affinity for the target antigen human FGFR2(β)b.

**Table 4. Affinity of antibodies for human FGFR2b protein**

| **Antibody name** | **ka (1/Ms)** | **kd (1/s)** | **KD (M)** | **Measured Rmax (RU)** |
|---|---|---|---|---|
| Ab-1 | 7.28E+04 | 1.30E-03 | 1.78E-08 | 111.9 |
| Ab-2 | 2.12E+05 | 4.91E-04 | 2.31E-09 | 101.0 |
| Ab-3 | 1.91E+05 | 2.24E-04 | 1.17E-09 | 104.7 |
| FPA144-hIgG1 | 1.27E+05 | 2.78E-04 | 2.18E-09 | 106.5 |
| mAbl | 8.35E+04 | 8.24E-04 | 9.87E-09 | 97.1 |
| mAb2 | 5.83E+04 | 9.50E-04 | 1.63E-08 | 89.7 |

### Example 2: Preparation of antibody-drug conjugates

### 2.1 Preparation/source of drug-linker compound

The structure of drug-linker 1 is shown below, and reference was made to Patent No. WO2023217227A1 (Example 39) for its preparation.

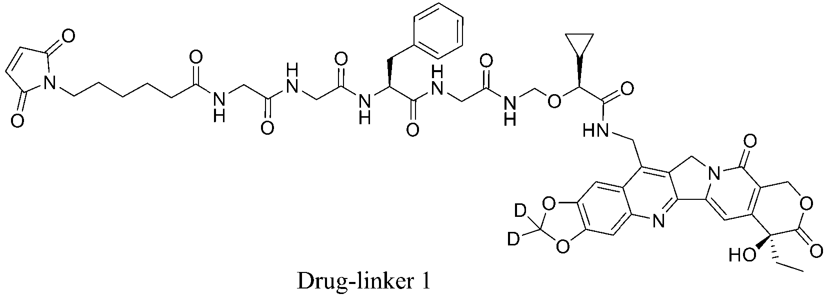

The structure of drug-linker 2 is shown below, and the compound was purchased from MedChemExpress (MCE) under Cat. No. HY-15575.

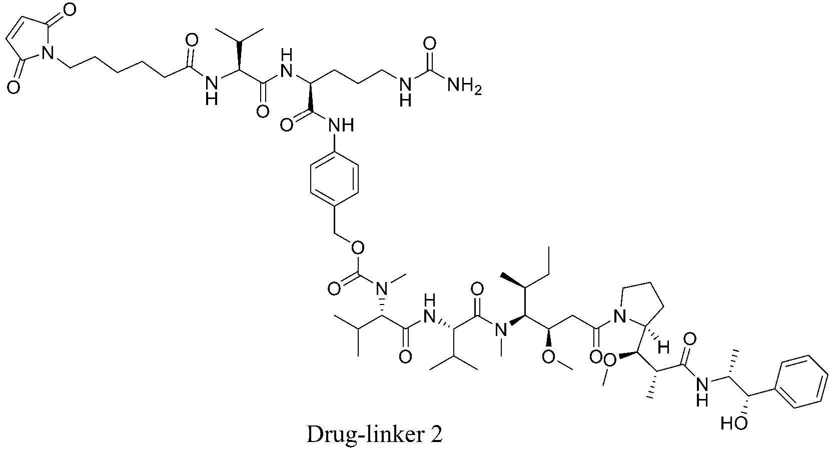

### 2.2 Conjugation of antibody to drug-linker

Tris(2-carboxyethyl)phosphine hydrochloride (TCEP) of 10-fold molar equivalent was added to a 2-10 mg/mL antibody solution (1× PBS, pH 7.4). The mixed solution was mixed well and incubated on a thermostatic metal shaker at 37 °C for 2 h, and then the drug-linker 1 compound described above of 15-fold molar equivalent was added. The reaction solution was mixed well and reacted at 25 °C for 4 h. After the reaction was completed, the mixture was subjected to buffer exchange by ultrafiltration 3 times with a 10 mM NaAC-HAc buffer (pH 5.5) to remove residual unreacted free small molecules, so as to afford the antibody-drug conjugate.

Tris(2-carboxyethyl)phosphine hydrochloride of 2.0- to 2.5-fold molar equivalent was added to a 2-10 mg/mL antibody solution (1× PBS, pH 7.4). The mixed solution was mixed well and incubated on a thermostatic metal shaker at 37 °C for 3 h, and then the drug-linker 2 compound described above of 15-fold molar equivalent was added. The reaction solution was mixed well and reacted at 25 °C for 16 h. After the reaction was completed, the mixture was subjected to buffer exchange by ultrafiltration 3 times with a 1× PBS buffer to remove residual unreacted free small molecules, so as to afford the antibody-drug conjugate.

### 2.3. Determination of purity and DAR value of ADCs

SEC purity analysis: The test protein sample was analyzed using the SEC-HPLC method to characterize the molecular size homogeneity of the recombinant protein and determine the purity of the recombinant protein. In this method, the HPLC instrument used was Agilent 1260, the chromatographic column was TSKgel G3000SWXL (purchased from Tosoh Bioscience), the mobile phase was 200 mM phosphate buffer, pH 7.0/isopropanol (Merck, 1.01040.4008) (v/v 9:1), the detection temperature was 25 °C, the flow rate was 0.5 mL/min, the detection wavelength was 280 nm, the loading amount of the target protein was 50 µg, and the analysis time was 40 min.

DAR value determination: The DAR value of the ADC molecule was determined using the ultrahigh-performance liquid chromatography-mass spectrometry (UHPLC-MS) method. Firstly, the test ADC molecule was treated with PNGase F (NEB #P0705L) to remove N glycosylation modification, then treated with dithiothreitol (DTT, Sigma #646563), incubated at 37 °C for 1 h for reduction into light and heavy chains, and then analyzed using a Thermo Vanquish UHPLC-Q Exactive Plus mass spectrometry system. Subsequently, 2 µg of the protein was injected into a Waters ACQUITY Protein BEH molecular exclusion chromatographic column, the mobile phase was an aqueous solution containing 0.1% formic acid, 0.05% TFA, and 25% acetonitrile, the flow rate was 0.2 mL/min, the analysis time was 30 min, the mass spectrometer was Thermo Q Exactive Plus, and the main parameters of the mass spectrometry were a spray voltage of 3.8 kV, a capillary heating temperature of 300 °C, a sheath gas flow rate of 35 arb, a precursor ion scanning range of 800-3000, etc. Finally, deconvolution processing was performed through the Respect algorithm using the mass spectrum data analysis software Biopharma Finder 4.1, and the molecular weight information of mass spectrum peaks of the light and heavy chains and the mass spectrum response signals of each component were separately calculated so as to calculate the DAR value of the test ADC sample, as shown in **Table 5.**

**Table 5. Preparation, DAR value, and purity of antibody-drug conjugates**

| Conjugate No. | Conjugation substrate | | DAR | SEC purity (monomer%) |
|---|---|---|---|---|
| | Antibody | Drug-linker | | |
| ADC-1 | FGFR2b-Ab-1 | Drug-linker 1 | 7.6~8.0 | Greater than 90% |
| ADC-2 | FGFR2b-Ab-2 | Drug-linker 1 | | |
| ADC-3 | FGFR2b-Ab-3 | Drug-linker 1 | | |
| ADC-4 | anti-FITC-hIgG 1 | Drug-linker 1 | | |
| ADC-5 | FGFR2b-Ab-1 | Drug-linker 2 | | |
| ADC-6 | FGFR2b-Ab-2 | Drug-linker 2 | 3.9~4.5 | Greater than 90% |
| ADC-7 | FGFR2b-Ab-3 | Drug-linker 2 | | |
| ADC-8 | anti-FITC-hIgG 1 | Drug-linker 2 | | |

### Example 3: Binding activity of anti-FGFR2b antibodies and FGFR2b-ADCs to tumor cells

KATOIII tumor cells with high human FGFR2b expression (purchased from Cobioer, Nanjing), SNU-16 tumor cells with medium-high expression (purchased from Cobioer, Nanjing), OVCAR3 tumor cells with medium-low expression (purchased from ATCC), and NUGC4 tumor cells with low expression (purchased from JCRB) were expanded in T-175 cell culture flasks until a confluence of 90%. The medium was completely discarded. The cells were washed once with PBS, treated with Versene (purchased from Gibco, Cat. No. 15040066), and collected. After counting, the cells were washed 2 times with PBS phosphate-buffered saline (purchased from Hyclone, Cat. No. SH30256.01), diluted to 2 × 10⁶ cells/mL, and added to a 96-well FACS reaction plate at 50 µL/well. 1% (v/v) fetal bovine serum (purchased from Excell Bio, Cat. No. FSP500) was added to the PBS phosphate-buffered saline as a flow cytometric (FACS) buffer, and the plate was centrifuged at 1500 rpm and 4 °C and washed 2 times. 100 µL of diluted antibody was added to each well, and the plates were incubated at 4 °C for 1 hour. After the plates were centrifuged and washed thrice with FACS buffer, 50 µL of Alexa Fluor 647 fluorescently labeled goat anti-human IgG (H+L) secondary antibody (purchased from Jackson Immuno, Cat. No. 109-605-088) was added to each well, and the plates were incubated at 4 °C for 1 hour. The plates were centrifuged and washed thrice with FACS buffer. The cells were suspended in 100 µL of FACS buffer and subjected to FACS (purchased from BD, Cat. No. FACS CantoII).

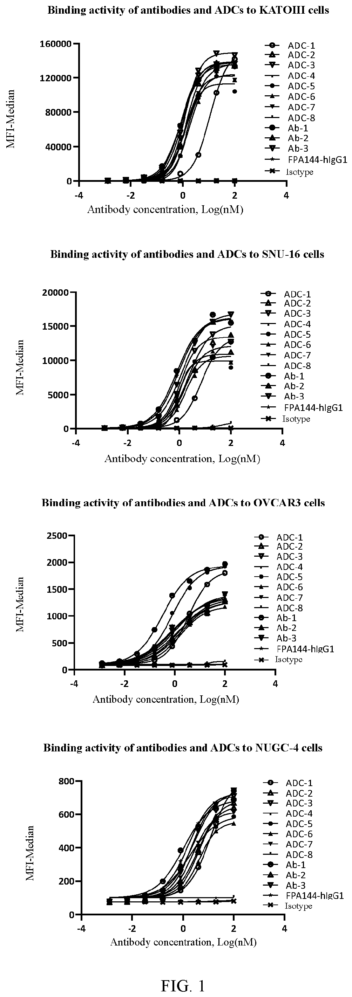

The detection results are shown in **Table 6** and FIG. 1. The binding activity of ADC-1 to tumor cells was lower than that of Ab-1, and ADC-2 and ADC-3 exhibited no significant changes in the binding activity to tumor cells; ADC-5, ADC-6, and ADC-7 exhibited no significant changes in the binding activity to tumor cells.

**Table 6. Binding activity of anti-FGFR2b antibodies and FGFR2b-ADCs to tumor cells**

| **Molecule name** | **Binding MFI** | | | | | | | |
|---|---|---|---|---|---|---|---|---|
| | **KATOIII** | | **SNU16** | | **OVCAR3** | | **NUGC-4** | |
| | **Emax** | **EC50 (nM)** | **Emax** | **EC50 (nM)** | **Emax** | **EC50 (nM)** | **Emax** | **EC50 (nM)** |
| ADC-1 | 142130 | 11.30 | 12745 | 7.09 | 1801 | 3.43 | 742 | 11.97 |
| ADC-5 | 121886 | 1.44 | 10551 | 0.96 | 1923 | 0.81 | 586 | 1.77 |
| Ab-1 | 134184 | 0.86 | 16684 | 0.80 | 1969 | 0.34 | 688 | 1.03 |
| ADC-2 | 137674 | 1.42 | 12646 | 1.75 | 1269 | 1.69 | 640 | 4.09 |
| ADC-6 | 128214 | 1.81 | 11307 | 1.77 | 1174 | 1.66 | 546 | 4.31 |
| Ab-2 | 134266 | 1.61 | 15604 | 2.34 | 1306 | 1.51 | 668 | 4.20 |
| ADC-3 | 148653 | 1.23 | 13810 | 1.12 | 1409 | 1.22 | 711 | 2.24 |
| ADC-7 | 125731 | 1.28 | 12016 | 0.98 | 1285 | 1.27 | 611 | 2.39 |
| Ab-3 | 136721 | 1.00 | 16719 | 1.36 | 1398 | 0.94 | 744 | 2.60 |
| FPA144-hIgG1 | 136991 | 0.87 | 15853 | 0.91 | 1373 | 0.80 | 737 | 1.64 |
| ADC-4 | 141 | No binding | 93 | No binding | 108 | No binding | 86 | No binding |
| ADC-8 | 457 | No binding | 756 | No binding | 147 | No binding | 104 | No binding |
| Isotype | 119 | No binding | 108 | No binding | 93 | No binding | 81 | No binding |

### Example 4: Endocytic effect of anti-FGFR2b antibodies

The culture medium for KATOIII cells (purchased from Cobioer, Nanjing, Cat. No. CBP60483) used in this study was RPMI 1640 (purchased from Gibco, Cat. No. 10491) + 10% FBS (purchased from Gibco, Cat. No. 10091-148). KATOIII cells were resuspended in a flow cytometric buffer (purchased from Biolegend, Cat. No. 420201) and adjusted to a cell density of 2 × 10⁶ cells/mL. The cell suspension was added to round-bottom 96-well plates (purchased from Corning, Cat. No. 3799) at 1 × 10⁵ cells/50 µL/well. 50 µL of antibodies serially 3-fold diluted from 100 nM were added, and the plates were incubated for 1 hour at 4 °C. After 1 hour, unbound antibodies were washed away, and the cells were resuspended in the flow cytometric buffer. The control group was let stand at 4 °C and the endocytosis group at 37 °C for 4 hours. After 4 hours, the plates were washed twice with PBS before Alexa Fluor 647 fluorescently labeled goat anti-human IgG Fcγ secondary antibody (purchased from Jackson Immuno, Cat. No. 109-605-098) was added. The plates were incubated at 4 °C for another 1 hour, washed twice with PBS, and subjected to FACS to determine the Alexa Fluor 647 fluorescent signal (MFI). The results were calculated and plotted using GraphPad Prism 9.0 software. Wells with the negative reference antibody Isotype were defined as the negative controls. Endocytosis efficiency = (control group MFI - endocytosis group MFI)/control group MFI × 100%.

The results are shown in **Table 7.** The negative control Isotype exhibited no significant endocytic activity in KATOIII cells at the saturated concentration of 100 nM, and the 3 anti-FGFR2b antibodies Ab-1, Ab-2, and Ab-3 of the present disclosure exhibited a certain endocytic activity in KATOIII cells (endocytic rate: 40% to 43.5%). The endocytic activity of the anti-FGFR2b monoclonal antibodies of the present disclosure was superior to that of the reference antibody FPA144-hIgG1 (endocytosis rate: 30.5%).

**Table 7. Endocytic activity of anti-FGFR2b antibodies in KATOIII cells**

| **Antibody** | **Endocytic rate (%)** |
|---|---|
| Ab-1 | 43.5 |
| Ab-2 | 42.5 |
| Ab-3 | 40 |
| FPA144-hIgG1 | 30.5 |
| Isotype | No endocytosis |

### Example 5. Inhibitory effects of ADCs on in vitro proliferation of tumor cells

In this experiment, the medium for KATOIII cells (purchased from Cobioer, Nanjing, Cat. No. CBP60483) was RPMI 1640 + 10% FBS, the medium for SNU16 cells (purchased from Cobioer, Nanjing, Cat. No. CBP60502) was RPMI 1640 + 10% FBS, and the medium for OVCAR3 cells (purchased from ATCC, Cat. No. HTB-161) was 1640 + 20% FBS + 10 µg/mL insulin (purchased from Solarbio, Cat. No. 40112ES25).

In this experiment, KATOIII is a cell line with high FGFR2b expression, SNU16 is a cell line with medium-high FGFR2b expression, and OVCAR3 is a cell line with medium FGFR2b expression. KATOIII, SNU16, and OVCAR3 cells were digested with Versene (purchased from Gibco, Cat. No. 15040066) and then resuspended in their corresponding media, and the cell density was adjusted. KATOIII, SNU16, and OVCAR3 cells were seeded in 96-well plates (purchased from Corning, Cat. No. 3610) at 5 × 10³ cells/90 µL and cultured overnight in an incubator at 37 °C with 5% CO₂. The next day, the antibody-drug conjugate was serially diluted with the complete medium according to the experimental design, and 10 µL of the diluted ADC was transferred to the corresponding well plate. The cell plate was incubated in an incubator at 37 °C with 5% CO₂ for 5 days. After 5 days, 100 µL of CellTiter-Glo detection reagent (purchased from Promega, Cat. No. G9243, see the product instructions for use) was added to each well, and the fluorescence values were read on an Envision instrument (purchased from PerkinElmer, Cat. No. Envision 2105) to measure the cell viability.

A positive control group and a negative control group were set as 0% and 100% killing controls, respectively. No test compounds were added in the positive control group, and other operations were consistent with those in the experimental group. No cells were added in the negative control group, with only the culture medium in the same volume added, and other operations were consistent with those in the experimental group. The formula for calculating the cell killing rate: cell killing rate = ((positive control - sample well reading)/(positive control - negative well reading)) × 100%. The results were calculated and plotted using GraphPad Prism 9.0 software.

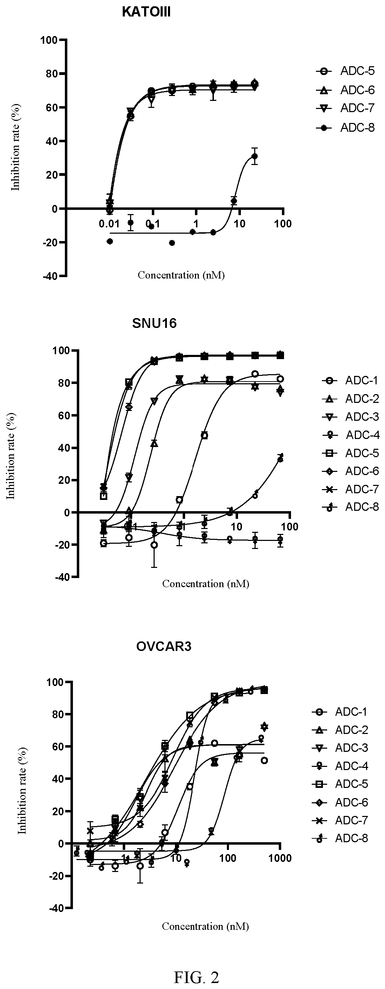

The results are shown in **Table 8** and FIG. 2. ADC drugs carrying different toxins all exhibited good proliferation inhibition activity against FGFR2b-expressing tumor cells *in vitro,* with killing activity IC50 in positive correlation with the FGFR2b expression.

**Table 8. Inhibitory effects of ADCs on proliferation of tumor cells**

| **ADC name** | **KATOIII** | | **SNU16** | | **OVCAR3** | |
|---|---|---|---|---|---|---|
| | **IC50 (nM)** | **Emax (%)** | **IC50 (nM)** | **Emax (%)** | **IC50 (nM)** | **Emax (%)** |
| ADC-5 | 0.028 | 72 | 0.057 | 96 | 4 | 94 |
| ADC-6 | 0.021 | 72 | 0.066 | 97 | 10 | 98 |
| ADC-7 | 0.021 | 70 | 0.054 | 96 | 7.2 | 100 |
| ADC-8 | >22.22 | 31 | >66.67 | 35 | 29 | 92 |
| ADC-1 | Not available | Not available | 2.2 | 84 | 15 | 56 |
| ADC-2 | Not available | Not available | 0.27 | 80 | 2.6 | 61 |
| ADC-3 | Not available | Not available | 0.13 | 79 | 2.1 | 61 |
| ADC-4 | Not available | Not available | No killing | No killing | 94 | 65 |

### Example 6. In vivo efficacy of ADCs in gastric cancer tumor-bearing mice

FGFR2b-positive human gastric cancer cells SNU-16-#232 and KATO-III-#729 were selected to establish an in vivo model in mice (CB17-SCID, female, aged 6-8 weeks, Beijing Vital River Laboratory Animal Technology Co., Ltd.) for evaluating the *in vivo* anti-tumor efficacy of the candidate molecules.

### 6.1. Inhibition of ADCs against growth of human gastric cancer SNU-16-#232 tumor

The day of human gastric cancer cells SNU-16-#232 (primary cells, purchased from Cobioer, Nanjing) grafting was taken as day 0. On day 0, a proper number of expanded human gastric cancer cells SNU-16-#232 in the logarithmic growth phase (about 80% confluence; transferred to a fresh culture medium on the day before grafting) were collected and grafted. The cell suspension was added into a 50-mL centrifuge tube and centrifuged for 7 minutes at 300 g. The cells were resuspended in a proper amount of serum-free RPMI1640 medium (purchased from Gibco, Cat. No. 61870-036), and 500 µL of the cell suspension was used for counting on a cell counter (Beckman, SIC-TP-573). According to the counting results, the cells were adjusted to a density of 200 × 10⁶ cells/mL with a serum-free medium and transferred to SPF animal room on ice for grafting and modeling. The cell suspension was mixed with an equal volume of Matrigel matrix (purchased from Corning, Cat. No. 356237), and subcutaneously administered to mice at the right axilla at 100 µL.

After the grafting of gastric cancer cells SNU-16-#232, tumor growth monitoring was performed. When the mean tumor volume was about 100-150 mm³, mice with appropriate tumor volumes were selected and randomized into groups of 6 mice each. The tail vein administration of the corresponding drugs was performed according to the regimen, and the tumor volume and changes in the mouse body weight were measured. The specific administration regimen is shown in **Table 9,** where PBS was the negative control group, ADC-4 and ADC-8 were isotype control antibodies, and ADC-2, ADC-3, ADC-5, ADC-6, and ADC-7 were ADC candidate molecules. The ADC candidate molecules were given at the same mass dose per unit body weight as the isotype control antibodies. Molecules with maytansinoid structures are generally more toxic than molecules with camptothecin structures when they are used to prepare antibody-drug conjugates, and maytansinoid ADCs are used at lower doses than camptothecin ADCs in clinical practice. Therefore, in the design of experiments, the dose of molecules having the drug-linker 2 structure (see Example 2) was lower than the dose of ADCs having the drug-linker 1 structure.

The tumor inhibition rate was calculated as follows: tumor inhibition rate (%) = [1 - (Vt (treatment group) - V0 (treatment group))/(Vt (isotype control group) - V0 (isotype control group))] × 100%, where V0 is the mean tumor volume at the time of grouping, and Vt is the mean tumor volume at a certain measurement after treatment.

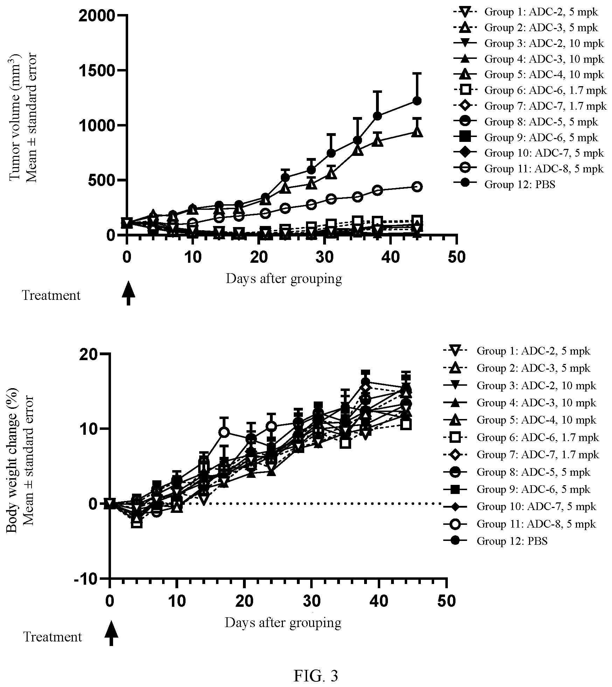

The results are shown in **Table 10** and FIG. 3. On day 44 after treatment, all the groups exhibited relatively good efficacy, and there were no significant abnormal changes in mouse body weight during the treatment period. ADC-8 exhibited more potent non-specific killing than ADC-4, thus suggesting a relatively smaller effective window for molecules with the drug-linker 2 structure.

**Table 9. Regimen of in vivo efficacy study of ADCs in human gastric cancer SNU-16-#232 tumor-bearing mice**

| **Group** | **Treatment** | **Dose (mg/kg, mpk)** | **Route and frequency of administration** |
|---|---|---|---|
| Group 1 | ADC-2 | 5.0 | |
| Group 2 | ADC-3 | 5.0 | |
| Group 3 | ADC-2 | 10.0 | |
| Group 4 | ADC-3 | 10.0 | |
| Group 5 | ADC-4 | 10.0 | |
| Group 6 | ADC-6 | 1.7 | Single dose via tail vein |
| Group 7 | ADC-7 | 1.7 | |
| Group 8 | ADC-5 | 5.0 | |
| Group 9 | ADC-6 | 5.0 | |
| Group 10 | ADC-7 | 5.0 | |
| Group 11 | ADC-8 | 5.0 | |
| Group 12 | PBS | - | |

**Table 10. In vivo efficacy results of ADCs in human gastric cancer SNU-16-#232 tumor-bearing mice**

| **Group** | **Treatment** | **Dose (mpk)** | **Day 44 post-treatment** | | | | | |
|---|---|---|---|---|---|---|---|---|
| | | | **Body weight change (%)** | **Tumor volume (mm³)** | **Tumor inhibitio n (%)** | **Significance** | **P value** | **Number of complete responses** |
| Group 1 | ADC-2 | 5 | 12.66 | 53.87 | 106.80 | **** | <0.000 1 | 0/6 |
| Group 2 | ADC-3 | | 14.87 | 83.05 | 103.74 | **** | <0.000 1 | 0/6 |
| Group 3 | ADC-2 | 10 | 15.56 | 20.64 | 110.99 | **** | <0.000 1 | 1/6 |
| Group 4 | ADC-3 | | 11.82 | 16.97 | 111.36 | **** | <0.000 1 | 2/6 |
| Group 5 | ADC-4 | | 12.36 | 941.00 | 25.31 (vs. group 12) | vs. group 12*** | 0.0009 | 0/6 |
| Group 6 | ADC-6 | 1.7 | 10.59 | 131.72 | 93.92 | **** | <0.000 1 | 0/6 |
| Group 7 | ADC-7 | | 14.85 | 122.55 | 96.31 | **** | <0.000 1 | 0/6 |
| Group 8 | ADC-5 | 5 | 13.33 | 71.85 | 112.25 | **** | <0.000 1 | 0/6 |
| Group 9 | ADC-6 | | 13.21 | 99.30 | 104.23 | **** | <0.000 1 | 0/6 |
| Group 10 | ADC-7 | | 11.40 | 90.51 | 106.10 | **** | <0.000 1 | 0/6 |
| Group 11 | ADC-8 | | 15.11 | 440.45 | 70.38 (vs. group 12) | vs. group 12**** | <0.000 1 | 0/6 |
| Group 12 | PBS | - | 15.48 | 1222.73 | N/A | N/A | N/A | 0/6 |

### 6.2. Inhibition of ADCs against growth of human gastric cancer KATOIII-#729 tumor

The day of human gastric cancer cells KATOIII-#729 (primary cells, purchased from Cobioer, Nanjing) grafting was taken as day 0. On day 0, a proper number of expanded human gastric cancer cells KATOIII-#729 in the logarithmic growth phase (about 80% confluence; transferred to a fresh culture medium on the day before grafting) were collected and grafted. The culture medium in the cell culture flask was discarded. The cells were washed twice with phosphate-buffered saline (PBS, purchased from Hyclone, Cat. No. SH30256.01). An appropriate amount of 0.25% pancreatin (purchased from Gibco, Cat. No. 25200-072) was added, and the flask was gently shaken to ensure the pancreatin evenly covered the cell surface. The system was placed in a 37 °C environment for 5 minutes of digestion, and a complete medium containing 10% of fetal bovine serum (FBS, purchased from Gibco, Cat. No. 10091-148/2418958P) was added to terminate the digestion reaction. The cells adhered to the bottom of the flask were gently detached by pipetting. The digested cell suspension was added into a 50-mL centrifuge tube and centrifuged for 5 minutes at 350 g. The cells were resuspended in a proper amount of serum-free RPMI1640 medium and filtered through a 70 µm sieve. 500 µL of the cell suspension was used for counting on a cell counter. According to the counting results, the cells were adjusted to a density of 100 × 10⁶ cells/mL with a serum-free medium and transferred to SPF animal room on ice for grafting and modeling. The cell suspension was mixed with an equal volume of Matrigel matrix, and subcutaneously administered to mice at the right axilla at 200 µL.

After the grafting of gastric cancer cells KATOIII-#729, tumor growth monitoring was performed. When the mean tumor volume was about 200 mm³, mice with appropriate tumor volumes were selected and randomized into groups of 6 mice each. The tail vein administration of the corresponding drugs was performed according to the regimen, and the tumor volume and changes in the mouse body weight were measured. The specific administration regimen is shown in **Table 11,** where PBS was the negative control group, and ADC-4 and ADC-8 were different isotype control antibodies.

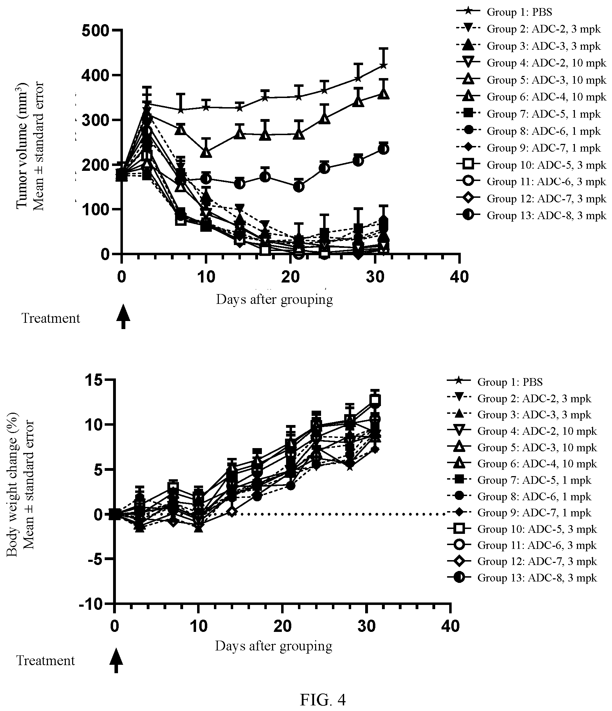

The results are shown in **Table 12** and FIG. 4. All the candidate molecules exhibited relatively good efficacy, with no significant differences between molecules. ADC-8 exhibited more potent non-specific killing than ADC-4, thus suggesting a relatively smaller effective window for molecules with the drug-linker 2 structure.

**Table 11. Regimen of in vivo efficacy study of ADCs in human gastric cancer KATOIII-#729 tumor-bearing mice**

| **Group** | **Treatment** | **Dose (mpk)** | **Route and frequency of administration** |
|---|---|---|---|
| Group 1 | PBS | - | |
| Group 2 | ADC-2 | 3 | |
| Group 3 | ADC-3 | 3 | |
| Group 4 | ADC-2 | 10 | |
| Group 5 | ADC-3 | 10 | |
| Group 6 | ADC-4 | 10 | |
| Group 7 | ADC-5 | 1 | Single dose via tail vein |
| Group 8 | ADC-6 | 1 | |
| Group 9 | ADC-7 | 1 | |
| Group 10 | ADC-5 | 3 | |
| Group 11 | ADC-6 | 3 | |
| Group 12 | ADC-7 | 3 | |
| Group 13 | ADC-8 | 3 | |

**Table 12. In vivo efficacy results of ADCs in human gastric cancer KATOIII-#729 tumor-bearing mice**

| **Group** | **Treatment** | **Dose (mpk)** | **Day 31 post-treatment** | | | | | |
|---|---|---|---|---|---|---|---|---|
| | | | **Body weight change (%)** | **Tumor volume (mm³)** | **Tumor inhibition (%)** | **Significance** | **P value** | **Number of complete responses** |
| Group 1 | PBS | - | 7.29 | 421.97 | N/A | N/A | N/A | 0/6 |
| Group 2 | ADC-2 | 3 | 9.07 | 50.59 | 169.21 | **** | <0.000 1 | 0/6 |
| Group 3 | ADC-3 | | 9.84 | 43.04 | 172.09 | **** | <0.000 1 | 1/6 |
| Group 4 | ADC-2 | 10 | 8.82 | 18.25 | 186.82 | **** | <0.000 1 | 2/6 |
| Group 5 | ADC-3 | | 8.64 | 22.11 | 185.74 | **** | <0.000 1 | 2/6 |
| Group 6 | ADC-4 | | 9.18 | 359.00 | 23.91 (vs. group 1) | vs. group 1**** | <0.000 1 | 0/6 |
| Group 7 | ADC-5 | 1 | 9.65 | 68.07 | 296.13 | **** | <0.000 1 | 0/6 |
| Group 8 | ADC-6 | | 9.84 | 56.33 | 322.99 | **** | <0.000 1 | 0/6 |
| Group 9 | ADC-7 | | 7.26 | 79.13 | 283.92 | **** | <0.000 1 | 0/6 |
| Group 10 | ADC-5 | 3 | 12.73 | 12.09 | 402.21 | **** | <0.000 1 | 4/6 |
| Group 11 | ADC-6 | | 10.61 | 10.71 | 408.07 | **** | <0.000 1 | 4/6 |
| Group 12 | ADC-7 | | 8.79 | 9.57 | 405.52 | **** | <0.000 1 | 4/6 |
| Group 13 | ADC-8 | | 12.38 | 235.24 | 77.29 (vs. group 1) | vs. group 1**** | <0.000 1 | 0/6 |

### Example 7: Efficacy verification of FGFR2b-ADCs

### 7.1. Preparation of reference FGFR2b-ADCs

The positive control FGFR2b ADCs were prepared with reference to Patent Nos. WO2022087243A1 and WO2022015656A1.

mAb1 and mAb2 IgGs were subjected to N297Q mutation to facilitate site-specific conjugation, and the antibody heavy chain sequences of the mAb1 and mAb2 mutants are shown in **Table 13.** With reference to the expression and purification methods for the antibodies in section 1.1 of Example 1, antibodies mAb1(N297Q) and mAb2(N297Q) were obtained.

**Table 13. Sequences of reference anti-FGFR2b antibody IgGs after N297Q mutation**

| **Sequence name** | SEQ ID NO. | **Amino acid sequence** |
|---|---|---|
| mAb1(N297Q) heavy chain | 35 | QVQLVESGGGVVQPGRSLRLSCAASGFTFSSYGMHWVRQAPG KGLEWVAVIWYDGSNKYYADSVKGRFTISRDNSKNTLYLQMNS LRAEDTAVYYCAREESSASGVDYWGQGTLVTVSSASTKGPSVFP LAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPC PPCPAPEFLGGPSVFLFPPKPKDTLMISRTPEVTCVVVDVSQEDPEV QFNWYVDGVEVHNAKTKPREEQFQSTYRVVSVLTVLHQDWLNGKE YKCKVSNKGLPSSIEKTISKAKGQPREPQVYTLPPSQEEMTKNQVSL TCLVKGFYPSDIAVEWESNGQPENNYKTTPPVLDSDGSFFLYSRLTV DKSRWQEGNVFSCSVMHEALHNHYTQKSLSLSLGK |
| mAb1 light chain | 30 | EIVLTQSPGTLSLSPGERATLSCRASQSVSTRYLAWYQQKRGQAP RLLIYGASSRATGIPDRFSGSGSGTDFTLTISRLEPEDFAVYYCQE YGSSSITFGQGTRLEIKRTVAAPSVFIFPPSDEQLKSGTASVVCLLNN FYPREAKVQWKVDNALQSGNSQESVTEQDSKDSTYSLSSTLTLSKAD YEKHKVYACEVTHQGLSSPVTKSFNRGEC |
| mAb2(N297Q) heavy chain | 36 | QVQLVESGGGVVQPGKSLRLSCAASGFTFSNYGMHWVRQTPV KGLEWVTLIWYDGSNKYYTDSVKGRFTISRDNSKNTLYLQMNS LRSEDTAVYYCAREGNWNYGHAFDIWGQGTMVTVSSASTKGPS VFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYG PPCPPCPAPEFLGGPSVFLFPPKPKDTLMISRTPEVTCVVVDVSQED PEVQFNWYVDGVEVHNAKTKPREEQFQSTYRVVSVLTVLHQDWLN GKEYKCKVSNKGLPSSIEKTISKAKGQPREPQVYTLPPSQEEMTKNQ VSLTCLVKGFYPSDIAVEWESNGQPENNYKTTPPVLDSDGSFFLYSR LTVDKSRWQEGNVFSCSVMHEALHNHYTQKSLSLSLGK |
| mAb2 light chain | 32 | DIQMTQSPSTLSASVGDRVTITCRASQNIGNWLAWYQQKPGKA PNLLIYKASTLESGVPSRFSGSGSGTEFTLTISSLQPDDFATYNCQ QYNSYSPTFGQGTKLEIKRTVAAPSVFIFPPSDEQLKSGTASVVCLL NNFYPREAKVQWKVDNALQSGNSQESVTEQDSKDSTYSLSSTLTLSK ADYEKHKVYACEVTHQGLSSPVTKSFNRGEC |

| | | |
|---|---|---|
| Note: antibody variable region + constant region (italic) | | |

The amino-azide linker AL1 (CAS: 134179-38-7; purchased from MCE, Cat. No. HY-WO21401) has the structure shown below.

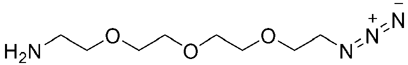

The structure of branched linker BL7 (CAS: 2253947-15-6) is shown below, and reference was made to Patent No. WO2018218004A1 for its preparation.

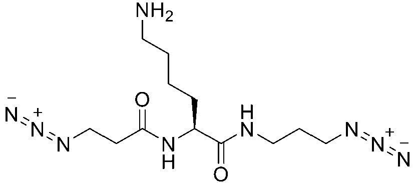

The structure of drug-linker 3 (CAS No. 2699066-62-9) is shown below, and the compound was purchased from MCE (Cat. No. HY-157407).

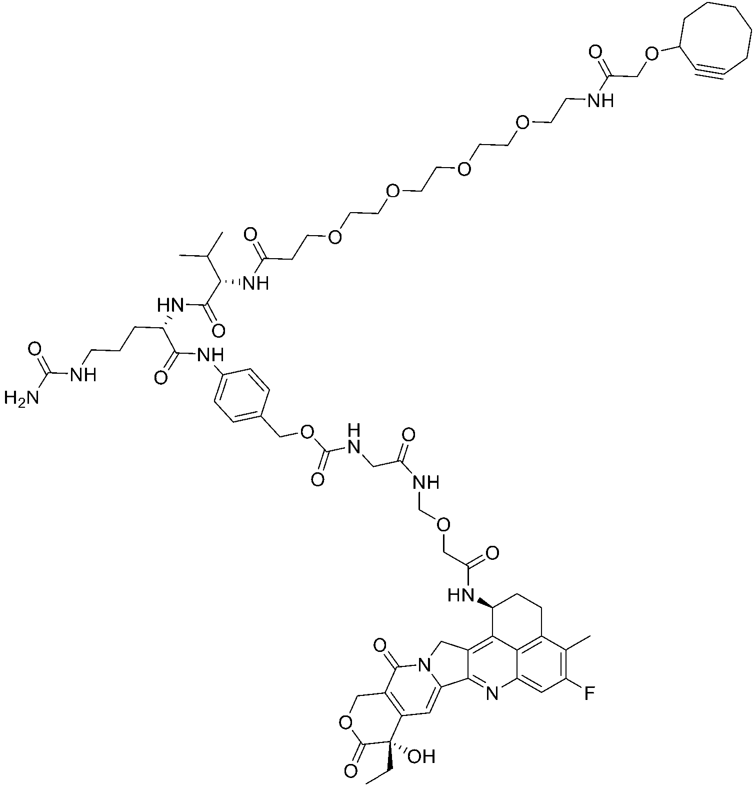

Transglutaminase (purchased from MCE, Cat. No. HY-P2962) was dissolved to 10 mg/mL in PBS, and the purified antibody sample was diluted to a concentration of 2 mg/mL with PBS. The dissolved enzyme was mixed with the antibody in a ratio of 10:1, and then linker AL1 or BL7 of 150-fold molar equivalent was added. After mixing, the mixture was left to stand overnight at 37 °C. Subsequently, excess enzyme and linker were removed on a Protein A column, and the sample was analyzed by MS. DAR4 and DAR8 molecules increased by 804 Da and 1232 Da, respectively.

Drug-linker 3 was conjugated to the linker to which the antibody had been linked using "click" chemistry. The antibody linked to AL1 or BL7 (1× PBS, pH 7.4) was mixed with greater than or equal to 10 molar equivalents of 10 mg/mL drug-linker 3 dissolved in DMSO, and the reaction solution contained 5%-15% (V/V) of organic solvent. The mixture was reacted overnight at room temperature. After the reaction, excess free small molecules were removed by cation exchange chromatography, and then the sample was dialyzed into a storage buffer using a dialysis cassette.

The SEC purity and DAR values of the ADC samples were obtained with reference to the purity and DAR value determination method for ADCs in Example 2.3, as shown in **Table 14.**

**Table 14. Preparation, DAR value, and purity of antibody-drug conjugates**

| Conjugate No. | Conjugation substrate | | | DAR | SEC purity (monomer%) |
|---|---|---|---|---|---|
| | Antibody | Linker | Drug-linker | | |
| ADC-9 | mAb1(297Q) | BL7 | Drug-linker 3 | 7.6~8.0 | Greater than 90% |
| ADC-10 | mAb2(297Q) | BL7 | Drug-linker 3 | | |
| ADC-11 | mAb 1(297Q) | AL1 | Drug-linker 3 | 3.9~4.5 | Greater than 90% |
| ADC-12 | mAb2(297Q) | AL1 | Drug-linker 3 | | |

### 7.2 Binding activity of anti-FGFR2b antibodies and FGFR2b-ADCs to tumor cells

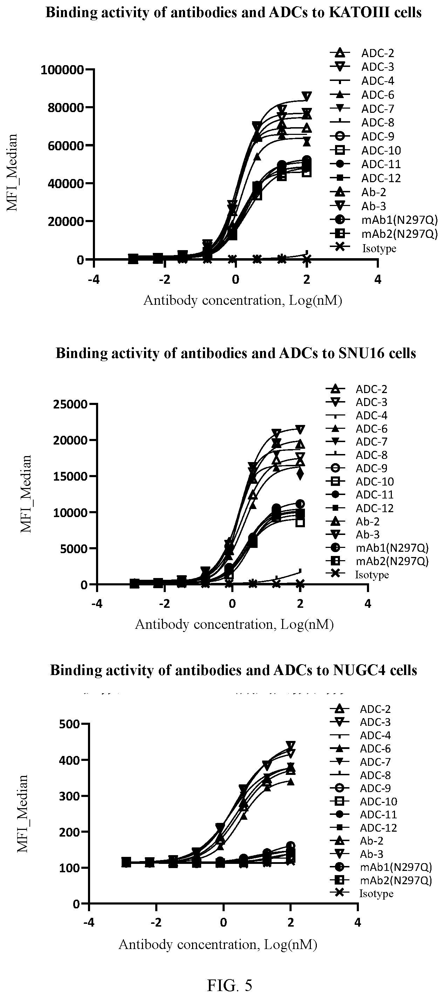

The binding activity of anti-FGFR2b antibodies and FGFR2b-ADCs to tumor cells was determined with reference to the method in Example 3. The results are shown in **Table 15** and **FIG. 5****.** The binding activity of the anti-FGFR2b antibodies and FGFR2b-ADCs of the present disclosure to tumor cells was higher than that of the reference anti-FGFR2b antibodies and FGFR2b-ADCs.

**Table 15. Binding activity of anti-FGFR2b antibodies and FGFR2b-ADCs to tumor cells**

| **Molecule name** | **Binding MFI** | | | | | |
|---|---|---|---|---|---|---|
| | **KATOIII** | | **SNU16** | | **NUGC4** | |
| | **Emax** | **EC50 (nM)** | **Emax** | **EC50 (nM)** | **Emax** | **EC50 (nM)** |
| ADC-2 | 69192 | 1.10 | 17234 | 1.90 | 372 | 2.80 |
| ADC-6 | 65342 | 1.41 | 16133 | 2.19 | 340 | 3.16 |
| Ab-2 | 76228 | 1.24 | 19552 | 1.71 | 380 | 2.43 |
| ADC-3 | 77210 | 1.12 | 19571 | 1.59 | 418 | 2.01 |
| ADC-7 | 68278 | 1.03 | 18002 | 1.36 | 382 | 1.58 |
| Ab-3 | 85830 | 1.39 | 21455 | 1.81 | 439 | 2.33 |
| ADC-9 | 48424 | 1.47 | 10360 | 2.42 | 146 | No binding |
| ADC-11 | 51107 | 1.68 | 10577 | 2.47 | 147 | No binding |
| mAb1(N297Q) | 52201 | 2.12 | 11171 | 3.26 | 161 | No binding |
| ADC-10 | 45686 | 1.47 | 9266 | 2.52 | 141 | No binding |
| ADC-12 | 48948 | 1.68 | 9484 | 2.79 | 138 | No binding |
| mAb2(N297Q) | 48702 | 2.08 | 9881 | 3.25 | 127 | No binding |
| ADC-4 | 217 | No binding | 176 | No binding | 122 | No binding |
| ADC-8 | 2767 | No binding | 1657 | No binding | 141 | No binding |
| Isotype | 233 | No binding | 120 | No binding | 118 | No binding |

### 7.3 Inhibitory effects of FGFR2b-ADCs on in vitro proliferation of tumor cells

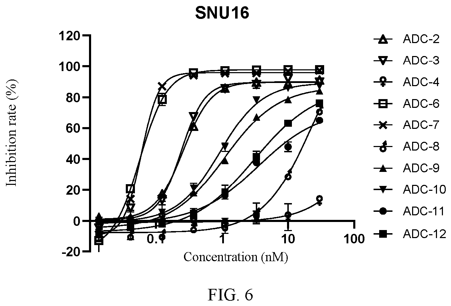

The inhibitory activity of FGFR2b-ADCs against the *in vitro* proliferation of tumor cells was determined with reference to the method in Example 5. The results are shown in **Table 16** and **FIG. 6****.** The inhibitory activity of the FGFR2b-ADCs of the present disclosure against the *in vitro* proliferation of tumor cells was higher than that of the reference FGFR2b-ADCs.

**Table 16. Inhibitory effects of ADCs on proliferation of tumor cells**

| **ADC name** | **SNU16** | |
|---|---|---|
| | **IC50 (nM)** | **Emax (%)** |
| ADC-2 | 0.26 | 91.0 |
| ADC-3 | 0.24 | 89.9 |
| ADC-4 | >30 | 12.8 |
| ADC-6 | 0.057 | 97.8 |
| ADC-7 | 0.060 | 97.3 |
| ADC-8 | 25.52 | 72.1 |
| ADC-9 | 1.11 | 84.1 |
| ADC-10 | 0.92 | 87.4 |
| ADC-11 | 3.50 | 65.1 |
| ADC-12 | 3.11 | 76.2 |

## Claims

1. An antibody-drug conjugate having a structural general formula of Pc-(L-D)ₙ or a pharmaceutically acceptable salt thereof, wherein
D is a cytotoxic drug;
L is a linker unit;
Pc is an antibody or an antigen-binding fragment thereof that specifically binds to FGFR2b; and
n is a real number of 1 to 16;
the antibody or the antigen-binding fragment thereof comprises a heavy chain variable region (VH) or/and a light chain variable region (VL), wherein the heavy chain variable region comprises an HCDR1, an HCDR2, and an HCDR3, or/and the light chain variable region comprises an LCDR1, an LCDR2, and an LCDR3, wherein the HCDR1-3 or/and the LCDR1-3 are selected from the following combinations:
(1) the HCDR1-3 are the sequences set forth in SEQ ID NOs. 9-11, or/and the LCDR1-3 are the sequences set forth in SEQ ID NOs. 12-14;
(2) the HCDR1-3 are the sequences set forth in SEQ ID NOs. 15-17, or/and the LCDR1-3 are the sequences set forth in SEQ ID NOs. 18-20;
(3) the HCDR1-3 are the sequences set forth in SEQ ID NOs. 21-23, or/and the LCDR1-3 are the sequences set forth in SEQ ID NOs. 24-26; or,
(4) the HCDR1-3 or/and the LCDR1-3 are sequences having at least 80% identity or having at most 3 insertion, deletion, or substitution mutations compared to any one CDR in the HCDR1-3 and the LCDR1-3 in any one of combinations (1)-(3); preferably, the at least 80% identity is 85%, 90%, 91%, 92%, 93%, 94%, 95%, 96%, 97%, 98%, 99%, or 100% identity.

2. The antibody-drug conjugate or the pharmaceutically acceptable salt thereof according to claim 1, wherein the antibody or the antigen-binding fragment thereof comprises a heavy chain variable region (VH) or/and a light chain variable region (VL), wherein the heavy chain variable region or/and the light chain variable region are selected from the following:
(1) the heavy chain variable region is the sequence set forth in SEQ ID NO. 1, or/and the light chain variable region is the sequence set forth in SEQ ID NO. 2;
(2) the heavy chain variable region is the sequence set forth in SEQ ID NO. 3, or/and the light chain variable region is the sequence set forth in SEQ ID NO. 4;
(3) the heavy chain variable region is the sequence set forth in SEQ ID NO. 5, or/and the light chain variable region is the sequence set forth in SEQ ID NO. 6; or,
(4) the heavy chain variable region or/and the light chain variable region are sequences having at least 80% identity or having at most 3 insertion, deletion, or substitution mutations compared to the heavy chain variable region or/and the light chain variable region in any one of combinations (1)-(3); preferably, the at least 80% identity is 85%, 90%, 91%, 92%, 93%, 94%, 95%, 96%, 97%, 98%, 99%, or 100% identity.

3. The antibody-drug conjugate or the pharmaceutically acceptable salt thereof according to claim 2, wherein the antibody or the antigen-binding fragment thereof comprises a heavy chain constant region sequence and/or a light chain constant region sequence; optionally, the heavy chain constant region and/or the light chain constant region are selected from an intact constant region sequence or a fragment thereof, and the constant region fragment comprises CH1, a hinge region, CH2, CH3, or Fc; optionally, the heavy chain constant region is selected from a human or murine IgG1, IgG2, IgG3, or IgG4 constant region, and the light chain constant region is selected from a human or murine κ constant region or λ constant region; optionally, the antibody or the antigen-binding fragment thereof comprises intact heavy and light chains, wherein the heavy chain consists of the VH and the heavy chain constant region, the heavy chain constant region having the sequence set forth in SEQ ID NO: 7, and the light chain consists of the VL and the light chain constant region, the light chain constant region having the sequence set forth in SEQ ID NO: 8.

4. The antibody-drug conjugate or the pharmaceutically acceptable salt thereof according to any one of claims 1 to 3, wherein the antibody or the antigen-binding fragment thereof is: (1) a chimeric antibody or a fragment thereof, (2) a humanized antibody or a fragment thereof, and/or (3) a fully human antibody or a fragment thereof;
preferably, the antibody or the antigen-binding fragment thereof is selected from a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, a naked antibody, a conjugated antibody, a humanized antibody, a fully human antibody, a Fab, a Fab', an F(ab')₂, an Fd, an Fv, an scFv, a diabody, or a single domain antibody.

5. The antibody-drug conjugate or the pharmaceutically acceptable salt thereof according to claim 4, wherein the antigen-binding fragment is selected from one or more of an F(ab)₂, a Fab', a Fab, an Fv fragment, an scFv, a nanobody, or an affibody.

6. The antibody-drug conjugate or the pharmaceutically acceptable salt thereof according to claim 1, wherein the cytotoxic drug is selected from a tubulin inhibitor, a DNA damaging agent, or a topoisomerase inhibitor, wherein the tubulin inhibitor includes, but is not limited to, a dolastatin, an auristatin, a maytansine, a tubulysin, and a cryptomycin, the DNA damaging agent includes, but is not limited to, a PBD drug, and the topoisomerase inhibitor includes, but is not limited to, a camptothecin drug.

7. The antibody-drug conjugate or the pharmaceutically acceptable salt thereof according to claim 6, wherein the cytotoxic drug is selected from a topoisomerase I inhibitor or an auristatin drug.

8. The antibody-drug conjugate or the pharmaceutically acceptable salt thereof according to claim 7, wherein the cytotoxic drug is selected from a compound of formula (D-I), 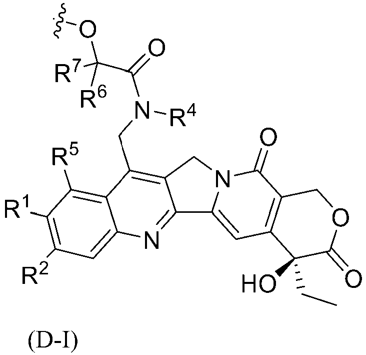 wherein
R¹ and R², together with the atoms linked thereto, form a 5- to 6-membered heterocyclyl, wherein the 5- to 6-membered heterocyclyl comprises 1 or 2 oxygen atoms as ring atoms, and the 5- to 6-membered heterocyclyl is optionally substituted with one or more D atoms;
R⁴ is selected from H or C₁-C₃ alkyl;
R⁵ is selected from H, halogen, CN, =O, OH, NH₂, or C₁-C₃ alkyl;
R⁶ is selected from H or C₁-C₃ alkyl;
R⁷ is selected from H, C₁-C₃ alkyl, or C₃-C₆ cycloalkyl, wherein the C₁-C₃ alkyl or the C₃-C₆ cycloalkyl is optionally substituted with D, halogen, CN, =O, OH, NH₂, or C₁-C₃ alkyl.

9. The antibody-drug conjugate or the pharmaceutically acceptable salt thereof according to claim 8,
wherein R¹ and R², together with the atoms linked thereto, form

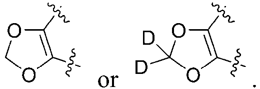

10. The antibody-drug conjugate or the pharmaceutically acceptable salt thereof according to claim 8, wherein R⁴ is selected from H.

11. The antibody-drug conjugate or the pharmaceutically acceptable salt thereof according to claim 8, wherein R⁵ is selected from H.

12. The antibody-drug conjugate or the pharmaceutically acceptable salt thereof according to claim 8, wherein R⁶ is selected from H.

13. The antibody-drug conjugate or the pharmaceutically acceptable salt thereof according to claim 8, wherein R⁷ is selected from cyclopropyl.

14. The antibody-drug conjugate or the pharmaceutically acceptable salt thereof according to any one of claims 8 to 13, wherein the compound of formula (D-I) is selected from

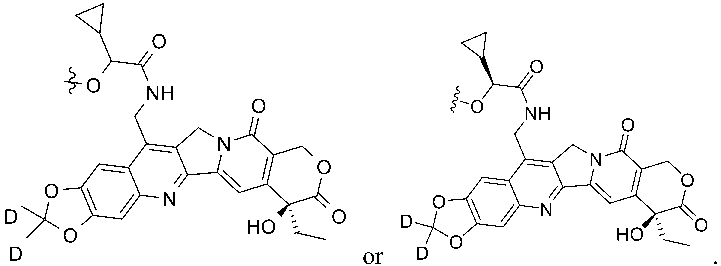

15. The antibody-drug conjugate or the pharmaceutically acceptable salt thereof according to any one of claims 7 to 14, wherein the cytotoxic drug is selected from

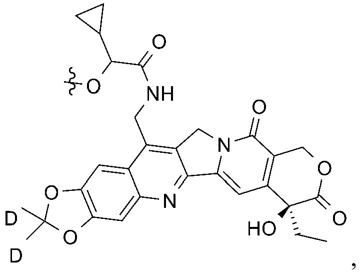

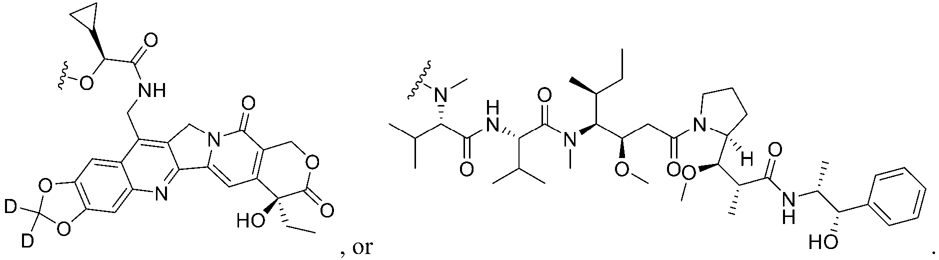

16. The antibody-drug conjugate or the pharmaceutically acceptable salt thereof according to claim 1, wherein the linker unit is selected from 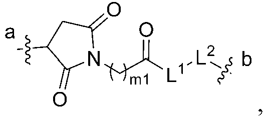 wherein m1 is selected from integers of 2 to 8, L¹ is selected from a peptide residue composed of 1 to 8 amino acids, and the peptide residue is further optionally substituted with one or more substituents selected from halogen, CN, =O, C₁-C₆ alkyl, OH, O(C₁-C₆ alkyl), NH₂, NH(C₁-C₆ alkyl), N(C₁-C₆ alkyl)₂, C₃-C₆ cycloalkyl, and 4- to 7-membered heterocyclyl; L² is selected from 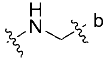 or 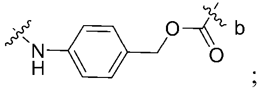 the end a of the linker unit is covalently linked to the antibody or the antigen-binding fragment thereof, and the end b is covalently linked to the cytotoxic drug.

17. The antibody-drug conjugate or the pharmaceutically acceptable salt thereof according to claim 16, wherein L¹ is selected from the peptide residue set forth in Val-Cit or Gly-Gly-Phe-Gly.

18. The antibody-drug conjugate or the pharmaceutically acceptable salt thereof according to claim 16, wherein m1 is 5.

19. The antibody-drug conjugate or the pharmaceutically acceptable salt thereof according to any one of claims 16 to 18, wherein the linker unit is 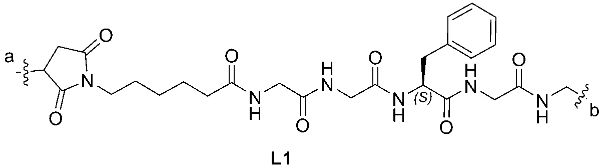 or 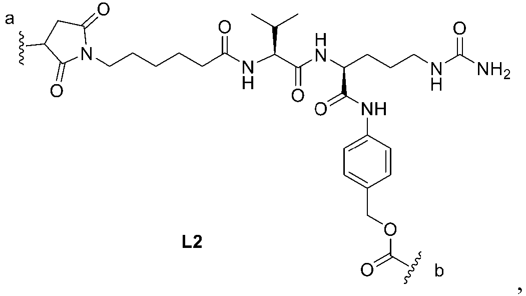 the end a of the linker unit is covalently linked to the antibody or the antigen-binding fragment thereof, and the end b of the linker unit is covalently linked to the cytotoxic drug.

20. The antibody-drug conjugate or the pharmaceutically acceptable salt thereof according to claim 1, wherein n is selected from real numbers of 1 to 16; for example, n is selected from real numbers of 2 to 12; for example, n is selected from real numbers of 4 to 10; for example, n is selected from real numbers of 3 to 9; for example, n is selected from real numbers of 4 to 8; for example, n is selected from real numbers of 6 to 8; for example, n is selected from real numbers of 3 to 5.

21. The antibody-drug conjugate or the pharmaceutically acceptable salt thereof according to claim 20, wherein n is selected from real numbers of 3 to 9; for example, n is 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0.

22. The antibody-drug conjugate or the pharmaceutically acceptable salt thereof according to claim 20, wherein n is selected from real numbers of 6 to 8; for example, n is 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0.

23. The antibody-drug conjugate or the pharmaceutically acceptable salt thereof according to claim 20, wherein n is selected from real numbers of 3 to 5; for example, n is 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0.

24. The antibody-drug conjugate or the pharmaceutically acceptable salt thereof according to any one of claims 1 to 23, wherein the antibody-drug conjugate or the pharmaceutically acceptable salt thereof is selected from the following compounds or pharmaceutically acceptable salts thereof: 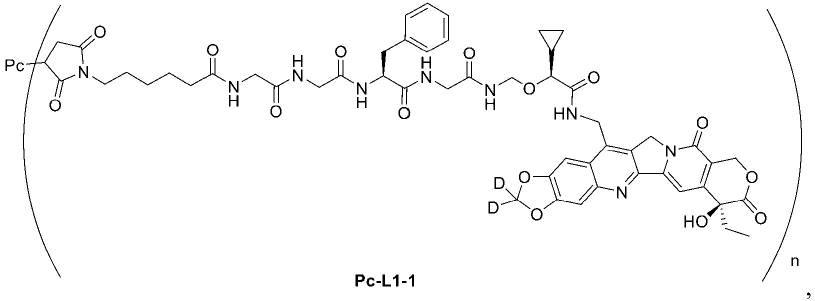 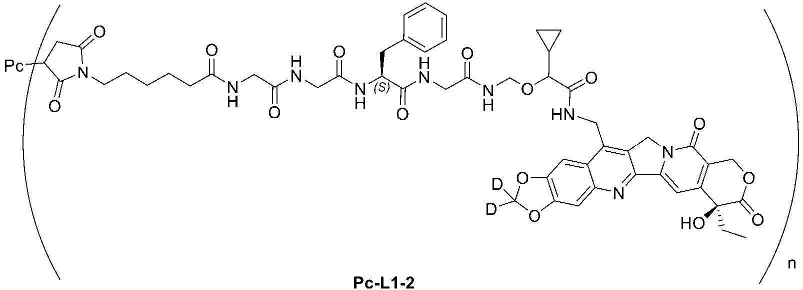 or 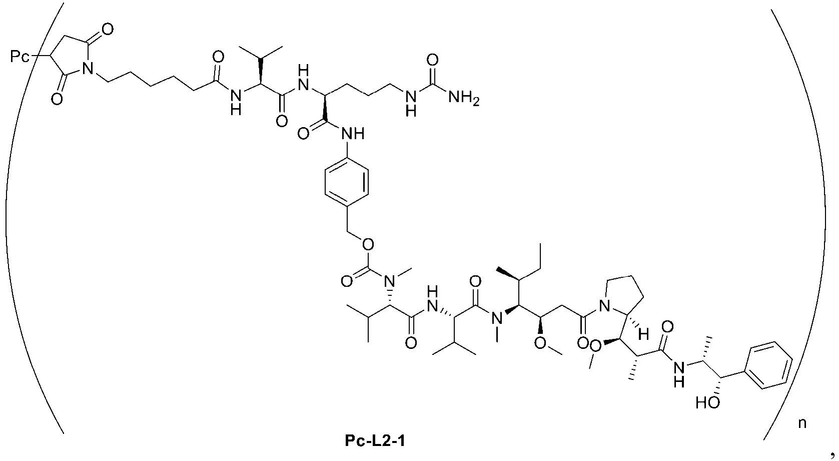 wherein Pc comprises the antibody or the antigen-binding fragment thereof described in any one of claims 1 to 5, and n includes the real numbers described in any one of claims 20 to 23.

25. An isolated nucleic acid molecule, wherein the nucleic acid molecule may be in the form of isolated nucleotides, deoxynucleotides, and/or ribonucleotides in any length, and the nucleic acid molecule encodes the antibody or the antigen-binding fragment thereof described in any one of claims 1 to 25.

26. An expression vector, comprising the isolated nucleic acid molecule according to claim 25.

27. An isolated host cell, comprising the nucleic acid molecule according to claim 25 or the expression vector according to claim 26, wherein preferably, the host cell is a eukaryotic cell or a prokaryotic cell; more preferably, the host cell is derived from a mammalian cell, a yeast cell, an insect cell, an *Escherichia coli* cell, and/or a *Bacillus subtilis* cell; more preferably, the host cell is selected from an Expi293 cell or a CHO cell.

28. A pharmaceutical composition, comprising the antibody-drug conjugate or the pharmaceutically acceptable salt thereof according to any one of claims 1 to 24 and a pharmaceutically acceptable excipient, wherein preferably, the pharmaceutical composition further comprises an additional therapeutic agent or a chemotherapeutic agent; preferably, the additional therapeutic agent comprises a PD-1/PD-L1 inhibitor.

29. A method for treating a tumor in a mammal, comprising: administering to the mammal, preferably a human, in need of the treatment a therapeutically effective amount of the antibody-drug conjugate or the pharmaceutically acceptable salt thereof according to any one of claims 1 to 24 or the pharmaceutical combination according to claim 28, wherein preferably, the method further comprises use of an additional therapeutic agent, such as an additional antibody, typically including a PD-1/PD-L1 inhibitor, or such as a chemotherapeutic agent; preferably, the tumor is an FGFR2b-expressing tumor; more preferably, the tumor is selected from the group consisting of: gastric cancer, squamous cell lung cancer, triple-negative breast cancer, endometrial carcinoma, ovarian cancer, pancreatic cancer, intrahepatic cholangiocarcinoma, and colorectal cancer.

30. Use of the antibody-drug conjugate or the pharmaceutically acceptable salt thereof according to any one of claims 1 to 24 or the pharmaceutical composition according to claim 28 in preparing a medicament for treating a tumor, wherein preferably, the pharmaceutical composition further comprises an additional therapeutic agent, such as an additional antibody, typically including a PD-1/PD-L1 inhibitor, or such as a chemotherapeutic agent; preferably, the tumor is an FGFR2b-expressing tumor; more preferably, the tumor is selected from the group consisting of: gastric cancer, squamous cell lung cancer, triple-negative breast cancer, endometrial carcinoma, ovarian cancer, pancreatic cancer, intrahepatic cholangiocarcinoma, and colorectal cancer.

31. Use of the antibody-drug conjugate or the pharmaceutically acceptable salt thereof according to any one of claims 1 to 24 or the pharmaceutical composition according to claim 28 in treating a tumor, wherein preferably, the pharmaceutical composition further comprises an additional therapeutic agent, such as an additional antibody, typically including a PD-1/PD-L1 inhibitor, or such as a chemotherapeutic agent; preferably, the tumor is an FGFR2b-expressing tumor; more preferably, the tumor is selected from the group consisting of: gastric cancer, squamous cell lung cancer, triple-negative breast cancer, endometrial carcinoma, ovarian cancer, pancreatic cancer, intrahepatic cholangiocarcinoma, and colorectal cancer.

32. The antibody-drug conjugate or the pharmaceutically acceptable salt thereof according to any one of claims 1 to 24 or the pharmaceutical composition according to claim 28 for use in treating a tumor, wherein preferably, the pharmaceutical composition further comprises an additional therapeutic agent, such as an additional antibody, typically including a PD-1/PD-L1 inhibitor, or such as a chemotherapeutic agent; preferably, the tumor is an FGFR2b-expressing tumor; more preferably, the tumor is selected from the group consisting of: gastric cancer, squamous cell lung cancer, triple-negative breast cancer, endometrial carcinoma, ovarian cancer, pancreatic cancer, intrahepatic cholangiocarcinoma, and colorectal cancer.
